(19) Europäisches Patentamt
European Patent Office
Office européen des brevets

(11) EP 1 265 911 B1

(12) **EUROPEAN PATENT SPECIFICATION**

(45) Date of publication and mention of the grant of the patent:
**21.05.2008 Bulletin 2008/21**

(21) Application number: **01918812.7**

(22) Date of filing: **16.03.2001**

(51) Int Cl.:
*C07J 41/00* (2006.01) *A61K 31/57* (2006.01)
*C07J 31/00* (2006.01) *C07J 7/00* (2006.01)
*C07J 5/00* (2006.01) *C07J 9/00* (2006.01)
*A61K 31/575* (2006.01) *A61P 5/36* (2006.01)

(86) International application number:
**PCT/US2001/008681**

(87) International publication number:
**WO 2001/074840 (11.10.2001 Gazette 2001/41)**

(54) **17-ALPHA-SUBSTITUTED-11-BETA-SUBSTITUTED-4-ARYL AND 21-SUBSTITUTED 19-NORPREGNADIENEDIONE AS ANTIPROGESTATIONAL AGENTS**

17-ALPHA-SUBSTITUIERTE-11-BETA-SUBSTITUIERTE-4-ARYL UND 21-SUBSTITUIERTE 19-NORPREGNADIENEDIONE MIT ANTIGESTAGENER AKTIVITÄT

MODIFICATION STRUCTURALE DE 19-NORPROGESTERONE I: 17-ALPHA-SUBSTITUE-11-BETA-SUBSTITUE-4-ARYLE ET 21-SUBSTITUE 19-NORPREGNADIENEDIONE EN TANT QUE NOUVEAUX AGENTS ANTIPROGESTATIFS

(84) Designated Contracting States:
**AT BE CH CY DE DK ES FI FR GB GR IE IT LI LU MC NL PT SE TR**

(30) Priority: **17.03.2000 US 526855**

(43) Date of publication of application:
**18.12.2002 Bulletin 2002/51**

(73) Proprietor: **THE GOVERNMENT OF THE UNITED STATES OF AMERICA, as represented by THE SECRETARY, DEPARTMENT OF HEALTH AND HUMAN SERVICES**
**Rockville, MD 20852 (US)**

(72) Inventors:
- **KIM, Hyun, K.**
  **Bethesda, MD 20817 (US)**
- **BLYE, Richard, P.**
  **Highland, MD 20777 (US)**
- **RAO, Pemmaraju, N.**
  **San Antonio, TX 78228 (US)**
- **CESSAC, James, W.**
  **San Antonio, TX 78230 (US)**
- **ACOSTA, Carmie, K.**
  **San Antonio, TX 78213 (US)**
- **SIMMONS, Anne, Marie**
  **San Antonio, TX 78201 (US)**

(74) Representative: **Woods, Geoffrey Corlett**
**J.A. KEMP & CO.**
**Gray's Inn**
**14 South Square**
**London WC1R 5JJ (GB)**

(56) References cited:
**WO-A-00/34306** **WO-A-89/12448**
**WO-A-97/41145** **WO-A-99/45022**

- **RAO P N ET AL: "11beta-Substituted 13beta-ethyl gonane derivatives exhibit reversal of antiprogestational activity" STEROIDS: STRUCTURE, FUNCTION, AND REGULATION, US,ELSEVIER SCIENCE PUBLISHERS, NEW YORK, NY, vol. 63, no. 1, 1998, pages 50-57, XP004103060 ISSN: 0039-128X**
- **RAO P N ET AL: "New 11beta-aryl-substituted steroids exhibit both progestational and antiprogestational activity" STEROIDS: STRUCTURE, FUNCTION, AND REGULATION, US,ELSEVIER SCIENCE PUBLISHERS, NEW YORK, NY, vol. 63, no. 10, 1 October 1998 (1998-10-01), pages 523-530, XP004140834 ISSN: 0039-128X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 265 911 B1

## Description

### FIELD OF THE INVENTION

The present invention relates generally to the field of steroids and, in particular, to novel 17-α-substituted, 11-β-substituted-4-aryl and 21-substituted 19-norpregnadienedione analogs which possess potent antiprogestational activity with minimal antiglucocorticoid activity.

### BACKGROUND OF THE INVENTION

There have been numerous attempts over the past few decades to prepare steroids with antihormonal activity. These have been reasonably successful where antiestrogens and antiandrogens are concerned. However, the discovery of effective antiprogestational and antiglucocorticoid steroids has proved to be a formidable task for the steroid chemist. It has been generally recognized for some years, however, that antiprogestational steroids would find wide applicability in population control, while antiglucocorticoids would be extremely valuable in the treatment of, for example, Cushing's syndrome and other conditions characterized by excessive endogenous production of cortisone. In the last decade, largely through the efforts of Teutsch, *et al.* of the Roussel-Uclaf group in France, a new series of 19-nortestosterone derivatives has been synthesized with strong affinity for the progesterone and glucocorticoid receptors and with marked antiprogestational and antiglucocorticoid activity *in vivo*. This important discovery revealed the existence of a pocket in the progesterone/glucocorticoid receptors that is able to accommodate a large 11β-substituent on selected 19-nortestosterone derivatives. By suitable selection of such a substituent, steroids with antihormonal properties were obtained.

The pioneering studies of Teutsch, *et al.* on the synthesis of antiprogestational and antiglucocorticoid steroids is summarized in a recent review article (G. Teutsch in Adrenal Steroid Antagonism. Ed. M. K. Agarwal, Walter de Gruyter and Co., Berlin, 1984. pp. 43-75) describing the work leading to the discovery of RU-38,486, the first steroid of this type selected for clinical development. RU-38,486 or mifepristone was found to be an effective antiprogestational/contragestative agent when administered during the early stages of pregnancy (IPPF Medical Bulletin 20; No. 5, 1986). In addition to these antiprogestational properties, mifepristone has very significant antiglucocorticoid activity and was successfully used by Nieman, et al., J. Clin. Endocrinology Metab., 61:536, (1985)) in the treatment of Cushing's syndrome. In common with the vast majority of steroidal hormone analogs, mifepristone additionally exhibits a range of biological properties. Thus, for example, it exhibits growth-inhibitory properties towards estrogen-insensitive T47Dco human breast cancer cells (Horwitz, Endocrinology, 116:2236, 1985). Experimental evidence suggests that the metabolic products derived from mifepristone contribute to its antiprogestational and antiglucocorticoid properties (Heikinheimo, et al., J. Steroid Biochem., 26:279 (1987)).

Ideally, for purposes of contraception, it would be advantageous to have compounds which possess antiprogestational activity without (or with minimal) antiglucocorticoid activity. Although there have been a number of attempts to modify the mifepristone structure in order to obtain separation of the antiprogestational activity from the antiglucocorticoid activity, this goal has not yet been fully achieved. As such, there remains a need in the art for the development of new steroids which possess antiprogestational activity with minimal antiglucocorticoid activity.

### SUMMARY OF THE INVENTION

The present invention provides new steroids which possess potent antiprogestational activity with minimal antiglucocorticoid activity. More particularly, the present invention provides compounds having the general formula:

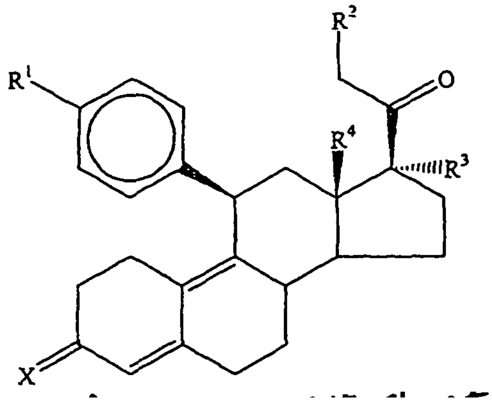

I

wherein: $R^1$ is $-N(CH_3)_2$; $R^4$ is methyl; X is = 0 ; and $R^2$ and $R^3$ are both $-OCH_3$, or $R^3$ is acetoxy and $R^2$ is a member selected from the group consisting of $-OCH=CH_2$ and $-OC(O)R^6$ wherein $R^6$ is $-CH_2OCH_3$.

As explained above, the compounds of the present invention possess potent antiprogestational activity with minimal antiglucocorticoid activity and, thus, they are suitable for long term use in the treatment of human endocrinologies or other conditions in steroid-sensitive tissues. Specific conditions for treatment include, but are not limited to, endometriosis (Kettel, L.M., et al., Fertil Steril, 56:402-407; Murphy, A.A., et al., Fertil Steril, 6:3761-766; Grow, D.R., et al., J. Clin. Endocrinol. Metab., 81:1933-1939.) uterine leiomyoma (Murphy, A.A., et al., Ibid.*;* Murphy, A.A., et al., J. Clin. Endocrinol. Metab., 76:513-517), uterine fibroid (Brogden, R.N., et al., Drugs, 45:384:409), meningioma (Brogden, R.N., et al., Ibid.*;* Poisson, M., et al., J. Neurooncol., 1:179-189; Carroll, R.S., et al., Cancer Res., 53:1312-1316; Mahajan, D.K. and London, S.N., Fertil Steril, 68:967-976 (1997)), and metastatic breast cancer (Brogden, R.N., *et al., Id.;* Rochefort, H., Trends in Pharmacol. Sci., 8:126-128; Horwitz, K.B., Endocr. Rev., 13:146-163 (1992) Mahajan, D.K. and London. S.N., *Id.*). Other uses include, but are not limited to, contraception (Wood, A.J.J., N. engl. J. Med., 329:404-412 (1993); Ulmann, A., et al., Sci. Amer., 262:42-48 (1990)), emergency postcoital contraceptive (Reel, J.R., et al., Contraception, 58:129-136 (1998)) and inducement of cervical ripening.

As such, in addition to providing compounds of Formula I, the present invention provides the use of the compounds of Formula I in the manufacture of a medicament to antagonize endogenous progesterone; to induce menses; to treat endometriosis; to treat dysmenorrhea; to treat endocrine hormone-dependent tumors (*e.g.*, breast cancer, uterine leiomyomas, *etc.*); to treat meningiomas; to treat uterine fibroids; to inhibit uterine endometrial proliferation; to induce cervical ripening; to induce labor; and for contraception.

Other features, objects and advantages of the invention and its preferred embodiments will become apparent from the detailed description which follows.

## BRIEF DESCRIPTION OF THE DRAWINGS

**Figures 1** through **11** illustrate the synthetic schemes used to prepare the compounds of Formula I and related compounds.

## DETAILED DESCRIPTION OF THE INVENTION AND PREFERRED EMBODIMENTS

The compounds of the present invention and related compounds can readily be synthesized in a variety of ways using modern synthetic organic chemistry techniques. Typically, the compounds are prepared using the synthetic schemes set forth in **Figures 1-11**. In general, there are five strategic steps that are useful in the synthesis of the antiprogestational agents. They are: (**1**) C21-substitution; (**2**) construction of the 17α-hydroxy-20-ketone pregnane side chain with the natural configuration via the SNAP reaction; (**3**) modification of the 17α-hydroxy moiety; (**4**) regiospecific synthesis of the epoxide and 1,4-conjugate grignard addition of a variety of 4-substituted aryl compounds; and (**5**) deketalization at C3 and 20 and concomitant dehydratration at C5. Each of these five strategic steps is described in greater detail hereinbelow. Moreover, a more detailed description of the synthetic protocols used to prepare the compounds of the present invention is set forth in the Example Section. It will be readily apparent to those of skill in the art that the particular steps, or combination of steps, used will vary depending on the compound being synthesized.

### 1. 21-Substitution

A number of different functional groups, such as F, Cl, Br, Me, hydroxy, alkoxy (*e.g.*, methoxy, ethoxy, *etc.*), acyloxy (*i.e.,* formyloxy, acetoxy, propionyloxy, *etc.*), cypionyloxy, methoxyacetoxy, and acylthio, have been introduced at C-21 of lead compound 17α-acetoxy-11β-(4-*N,N*-dimethylaminophenyl)-19-norpregna-4,9-diene-3,20-dione (CDB-2914 or C-21H or **69B**) using the synthetic schemes set forth in **Figures 1, 2** and **3**. For instance, a Silicon Nucleophilic Annulation Process (SNAP) on 17β-cyanohydrin (5) was used to prepare all of the 21-halogenated compounds with the exception of the 21-fluoro compound. This compound, however, was readily obtained by reacting the 21-mesylate with KF in acetonitrile in the presence of a crown ether. In addition, the 17α-acetoxy-21-ol compound (**41**) was obtained selectively from the ethoxyethylidenedioxy derivative (**18**) by means of buffered hydrolysis, whereas the 17α-ol-21-acetate derivative (**8**) was prepared from reacting the 21-halo compound with KOAc. It is interesting to note that both the 21-acetate and the 17α-acetate produced the 17α,21-diol (**9**) by a base catalyzed methanolosis. Thereafter, this 17α,21-diol was readily converted to the 17α,21-diacetate (**15**) by a mixed anhydride procedure. With regard to the synthesis of 17α-acetoxy-21-cypionate (**40**), the hydroxyl group at C-21 of the 17α,21-diol (**9**) was first converted to the corresponding cypionate (**39**) and then the 17α-OH group was acetylated. The 17α-acetoxy-21-thioacetate (**17**) was obtained by reaction of the 21-iodo compound generated *in situ* from the corresponding bromo compound (**7B**), with

potassium thioacetate followed by acetylation of the 17α-alcohol as shown in the synthetic scheme set forth in **Figure 1.**

Moreover, the 21-methyl analog (**28**) was prepared following the synthetic route set forth in **Figure 2**. The key reactions in this scheme are (1) the conversion of the 17α-cyanohydrin to the 17α-trimethylsilyloxy, 17α-aldehyde, and (2) the creation of the 21-methylprogesterone skeleton (**21** → **22**).

In addition, the 21-methoxy analog (**38**) was obtained following the synthetic scheme set forth in **Figure 3.** The key step in this scheme is the reaction of the 17α,21-diol protected at C-3 and C-20 with Meerwein's trimethyloxonium tetrafluoroborate salt in the presence of the sterically more hindered, less nucleophilic base, 1,8-bis(dimethylamino) naphthalene, as the proton sponge to selectively methylate the less-hindered 21-hydroxyl group. The subsequent epoxidation of the crude 21-methoxy compound (**34**) produced a 2:1 mixture of α and β epoxides as evidenced by $^1$H NMR. The crude epoxide (**35**) was subjected directly to the copper (I) catalyzed conjugate Grignard addition, assuming 66% of the crude epoxide was the desired - epoxide, hydrolysis and acetylation gave the 21-methoxy compound (**38**) with a purity of 98%. Following similar procedures, the 21-ethoxy compound (**46**) was obtained using triethyloxonium tetrafluoroborate salt. Treatment of the 21-acetete (**15**) and 21-methoxy compound (**38**) with hydroxylamine HCl followed by adjustment of the pH to pH 7 provided the desired 3-oximes, **47** and **48**, respectively, as a mixture of syn- and anti-isomers. Under these conditions, the sterically hindered C-20 ketone was intact as evidenced by IR spectroscopy.

In addition, using methods similar to those described above, additional functional groups, such as propionyloxy-(**126a**), 2-methoxyacetoxy- (**126b**), methylcarbonate (**126c**), 2-(*N,N*-dimethylamino)acetoxy-(**133**), and thiocyanato-(**138**) were readily synthesized *(see, e.g.,* **Figure 10 and 11**). Their synthetic methodology is straightforward. All of these compounds were derived from the previously prepared 17α,21-dihydroxy-11β-[4-(*N,N*-dimethylamino)phenyl]-19-norpregna-4,9-diene-3,20-dione (**9** in **Figure 1** or **124** in **Figure 11**). The C21-(1-ethenyl)oxy analog (**129**) was obtained from the C17α-acetoxy-21-ol (128) by reaction with ethyl vinyl ether in the presence of mercury(II) trifluoroacetate. Compound **128** was, in turn, obtained from hydrolysis of the 17α,21-cyclic ortho ester (**18** in **Figure 1** or **127** in **Figure 11**). Reaction of the C17α,21-diol (**9** in **Figure 1** or **124** in **Figure 11**) with methyl chloroformate in pyridine gave the methyl carbonate at C21(**125c**). Subsequent acetylation at C17 led to the target compound **126c** (*see,* **Figure 11**). Treatment of the C17α,21-diol (**9** or **124**) with methoxyacetyl chloride, followed by acetylation, provided **126b** (*see,* **Figure 11**). The synthesis of the 21-thiocyanato analog (**138**), which is illustrated in **Figure 11**, involved the preparation of the 21-mesylate (**136**), followed by thiocyanation at C21 (**137**) using the modified procedure of Abramson, H.N., et al. (J. Pharm. Sci. 65:765-768 (1976)). Subsequent acetylation at C17 led to the target compound (**138**). The 21-(*N, N*-dimethylamino)acetoxy (133) analog was obtained by preparing the 21-chloroacetate (**130**), acetylation of the 17α-OH (**131**) and converting the latter to the 21-iodoacetate (**132**) followed by the reaction of **132** with dimethylamine (*see,* **Figure 10**). This order of sequence did not result in hydrolysis of the 21-ester group. It is pointed out that an attempt to prepare the 21-iodoacetate (**132**) directly from the diol (**124**) was not as successful.

The 17α,21-diformate (**139**), which is illustrated in **Figure 10**, was synthesized by perchloric acid catalyzed formylation of the 17α,21-diol (**124**) following the procedure of Oliveto, E.P., et al. (J. Am. Chem. Soc., 77:3564-3567 (1955)). NMR analysis of this material indicated a 55:45 mixture of the 17α,21-diformate (**139**) resonating at 8.029 (s, C17-OC<u>H</u>O) and 8.165 ppm (s, C21-OC<u>H</u>O), respectively, and the 21-monoformate (**140**) at 8.172 ppm (s, C21-OC<u>H</u>O). Therefore, chromatographic separation was essential to obtain the pure 17α,21-diformate (**139**).

Syntheses of the 17α,21-dimethoxy derivatives (**113a**, **113b**, **133c** and **133d**) were achieved via oxidation at C-21 to afford the 21-hydroxy derivative (**107**) of the 17α-methoxy compound (**94**) following a modification of the procedure reported by Moriarty, R.M. et al., J. Chem. Soc. Chem. Commun., 641-642 (1981), and Velerio, et al., Steroids, 60: 268-271 (1995). Subsequent O-methylation provided the key 17α,21-dimethoxy intermediate (**108**) (*see,* **Figure 8**). Reduction of the 20-ketone (**108**) to the 20ξ-ol (**109**) followed by epoxidation at C5 and C10, copper (I) catalyzed conjugate Grignard addition to the 5α, 10α-epoxide (**110**), selective oxidation of the secondary alcohol, 20ξ-ol (**111**) using IBX to the 20-ketone (**112**), hydrolysis and acetylation, led to the target 17α,21-dimethoxy derivatives (**113**).

**2. Silicon Nucleophilic Annelation Process (SNAP)**

As described herein silylation of β-cyanohydrin ketal with halomethyldimethylsilyl chloride afforded the chloro- or bromomethyldimethylsilyl ether. The reductive SNAP reaction provided the 17α-hydroxy-20-ketopregnane side chain with the natural configuration at C17 (Livingston, D.A., et al., J. Am. Chem. Soc., 112:6449-6450 (1990); Livingston, D.A., Adv.Med Chem., 1:137-174 (1992); U.S. Patent No. 4,092,693, which issued to Livingston, D.A., et al. (May 1, 1990); U.S. Patent No. 4,977,255, which issued to Livingston, D.A., et al. (December 11, 1990). Alternatively, the formation of the halomethyldimethylsilyl ether, followed by treatment with lithium diisopropyl amide, provided the 21-substituted -17α-hydroxy-20-ketopregnanes.

**3. 17α-Substitution**

All 17α-esters illustrated in **Figures 4-11** were prepared from their 17α-hydroxy precursors. With the exception

of the 17α-formate (**69A**) and the 17α,21-diformate (**139**), all 17α-esters were also obtained via a mixed anhydride procedure (Carruthers, N.I. et al., J. Org. Chem., 57:961-965 (1992)),

17α-methoxy steroid (**93**) became available in large quantities from the 17α-hydroxydienedione (**92**) leading to a new series of antiprogestational agents, such as compounds **97** and **113**. Methylation of 17α-hydroxy group was most efficiently carried out using methyl iodide and silver oxide with acetonitrile as a cosolvent as described in the general procedure of Finch, et al. (J. Org. Chem., 40:206-215 (1975)). Other syntheses of 17α-methoxy steroids have been reported in the literature (*see, e.g.,* Numazawa, M. and Nagaoka, M., J. Chem. Soc. Commun., 127-128 (1983); Numazawa, M. and Nagaoka, M., J. Org. Chem., 50:81-84 (1985); Glazier, E.R., J. Org. Chem., 27:4397-4393 (1962).

The 17α-methoxymethyl compound (**91**) was obtained in 0.7% overall yield *via* the 14-step sequence illustrated in **Figure 5** starting from estrone methyl ether (**77**). No attempts were made to optimize the yield. The general strategy involved: (1) Construction of the 20-ketopregnane side chain; (2) Formation of the 17,20-enol acetate and subsequent alkylation with bromomethyl methyl ether; (3) Elaboration of the 3-ketal-5(10),9(11)-diene; (4) Epoxidation; (5) Conjugate Grignard addition; and (6) Hydrolysis.

### 4. 11β-Aryl-4-Substitution

The introduction of a variety of 4-substituted phenyl group at C11β into 19-norprogesterone requires the 5α, 10α-epoxide. Epoxidation of **2, 23, 34, 42, 50, 88, 94, 99, 109** and **119** has been known to be problematic *(see,* Wiechert, R. and Neef, G., J. Steroid Biochem., 27:851-858 (1987)). The procedure developed by Teutsch, G., et al. (Adrenal Steroid Antagonism (Agarwal, M.K., ed.), 43-75, Walter de Gruyter & Co., Berlin, N.Y. (1984)), *i.e.,* $H_2O_2$ and hexachloro or fluoroacetone, proved to be regioselective, but not highly stereoselective. A mixture of 5α,10α-epoxide and the corresponding 5β,10β-isomer was formed in approximately a 3:1 ratio. However, reduction of the C20-ketone (**108**) to the C20-ol (**109**) prior to epoxidation, resulted in a 9:1 ratio of the desired 5α,10α-epoxide.

Treatment of the 5α,10α-epoxides with 3-5 equivalents of Grignard reagents prepared from various 4-substituted aryl bromides *(see,* Yur'ev, Y.K., et al., Izvest. Akad. Nauk. S.S.S.R., Otdel Khim Nauk, 166-171 (CA 45: 10236f, (1951)); Wolfe, J.P. and Buchwald, S.L., J. Org. Chem., 62:6066-6068 (1997); Veradro, G., et al., Synthesis, 447-450 (1991); Jones, D.H., J. Chem. Soc. (C), 132-137 (1971); Detty et al., J. Am. Chem. Soc., 105:875-882 (1983), and Rao, P.N. et al., Steroids, 63:523-550 (1998)) in the presence of copper (I) chloride as a catalyst provided the desired 1β-4-substituted phenyl steroids. It is noted that 4-bromothioanisole was purchased from the Aldrich Chemical Co. (Milwaukee, Wisconsin). Evidence of the 11β-orientation of the 4-substituted phenyl substituent was shown by the upfiled shift of the C18 methyl group ($\delta$ = 0.273 - 0.484 ppm in $CDCl_3$), which is in agreement with Teutsch's observations (*see,* Teutsch, G. and Belanger, A., Tetrahedron Lett., 2051-2054 (1979)).

The presence of an unprotected 20-ketone resulted in low yields or in undesirable Grignard product mixtures. This was circumvented by reduction of the 20-ketone (analysis of this material by NMR indicated a single isomer; no further work was done for identification of this single isomer) prior to epoxidation and subsequent oxidation of the 20-alcohol by use of iodoxybenzoic acid (IBX) (Dess, D.B. and Martin, J.C., J. Org. Chem., 48:4155-4156 (1983); Frigerio, M. and Santagostino, M., Tetrahedron Letters, 35:8019-8022 (1994); and Frigerio, M. et al., J. Org. Chem., 60:7272-7276) after Grignard addition (*see,* **Figure 8**).

In case of **Figures 5** and **6**, the C3-ketone group was protected as a monoethyleneketal, and the C20-ketone was found to be intact when the Grignard reaction was followed during the multi-step procedures. For the syntheses of the 17α,21-diacetoxy derivatives (**Figure 7**), the strategy was to accomplish the conjugate addition prior to the SNAP reaction using the multi-step process described herein.

### 5. Deketalization

Deketalization with concomitant dehydration at C-5 in acidic media proceeded smoothly to provide the 4,9-diene-3,20-dione.

Quite surprisingly, the compounds of Formula I possess potent antiprogestational activity with minimal antiglucocorticoid activity. As a result of their antiprogestational activity, the compounds of Formula I can advantageously be used, *inter alia,* to antagonize endogenous progesterone; to induce menses; to treat endometriosis; to treat dysmenorrhea; to treat endocrine hormone-dependent tumors; to treat meningioma; to treat uterine leiomyonas, to treat uterine fibroids; to inhibit uterine endometrial proliferation; to induce labor; to induce cervical ripening, for hormone therapy; and for contraception.

More particularly, compounds having antiprogestational activity are characterized by antagonizing the effects of progesterone. As such, the compounds of the present invention are of particular value in the control of hormonal irregularities in the menstrual cycle, for controlling endometriosis and dysmenorrhea, and for inducing menses. In addition, the compounds of the present invention can be used to provide hormone therapy either alone or in combination with estrogenic substances in postmenopausal women, or in women whose ovarian hormone production is otherwise compromised.

Moreover, the compounds of the present invention can be used for control of fertility during the whole of the reproductive cycle. For long-term contraception, the compounds of the present invention can be administered either continuously or periodically depending on the dose. In addition, the compounds of the present invention are of particular value as postcoital contraceptives, for rendering the uterus inimical to implantation, and as "once a month" contraceptive agents.

A further important utility for the compounds of the present invention lies in their ability to slow down growth of hormone-dependent tumors and/or tumors present in hormone-responsive tissues. Such tumors include, but are not limited to, kidney, breast, endometrial, ovarian, and prostate tumors, *e.g.*, cancers, which are characterized by possessing progesterone receptors and can be expected to respond to the compounds of this invention. In addition, such tumors include meningiomas. Other utilities of the compounds of the present invention include the treatment of fibrocystic disease of the breast and uterine.

A given compound can readily be screened for its antiprogestational properties using, for example, the anti-McGinty test and/or the antiClauberg test described in the examples. In addition, a given compound can readily be screened for its ability to bind to the progesterone and/or glucocorticoid receptors or to inhibit ovulation using the assays described in the examples. Moreover, a given compound can readily be screened for its ability to inhibit tumor cell growth (*e.g.*, malignant tumor growth, *i.e.,* cancer) or to abolish tumorigenicity of malignant cells *in vitro* or *in vivo.* For instance, tumor cell lines can be exposed to varying concentrations of a compound of interest, and the viability of the cells can be measured at set time points using, for example, the alamar Blue® assay (commercially available from BioSource, International of Camarillo, California). Other assays known to and used by those of skill in the art can be employed.

The compounds according to the present invention can be administered to any warm-blooded mammal such as humans, domestic pets, and farm animals. Domestic pets include dogs, cats, *etc.* Farm animals include cows, horses, pigs, sheep goats, *etc.*

The amount of active ingredient that can be combined with a carrier material to produce a single dosage form will vary depending upon the disease treated, the mammalian species, and the particular mode of administration. For example, a unit dose of the steroid can preferably contain between 0.1 milligram and 1 gram of the active ingredient. A more preferred unit dose is between 0.001 and 0.5 grams. It will be understood, however, that the specific dose level for any particular patient will depend on a variety of factors including the activity of the specific compound employed; the age, body weight, general health, sex and diet of the individual being treated; the time and route of administration; the rate of excretion; other drugs which have previously been administered; and the severity of the particular disease undergoing therapy, as is well understood by those of skill in the area.

The compounds of the present invention can be administered by a variety of routes. Thus, those products of the invention that are active by the oral route can be administered in solutions, suspensions, emulsions, tablets, including sublingual and intrabuccal tablets, soft gelatin capsules, including solutions used in soft gelatin capsules, aqueous or oil suspensions, emulsions, pills, lozenges, troches, tablets, syrups or elixirs and the like. Products of the invention active on parenteral administration can be administered by depot injection, implants including Silastic TM and biodegradable implants, intramuscular and intravenous injections.

Compositions can be prepared according to any method known to the art for the manufacture of pharmaceutical compositions and such compositions can contain one or more agents selected from the group consisting of sweetening agents, flavoring agents, coloring agents and preserving agents. Tablets containing the active ingredient in admixture with nontoxic pharmaceutically acceptable excipients which are suitable for manufacture of tablets are acceptable. These excipients can be, for example, inert diluents, such as calcium carbonate, sodium carbonate, lactose, calcium phosphate or sodium phosphate, granulating and disintegrating agents, such as maize starch, or alginic acid; binding agents, such as starch, gelatin or acacia; and lubricating agents, such as magnesium stearate, stearic acid and talc. Tablets can be uncoated or, alternatively, they can be coated by known methods to delay disintegration and adsorption in the gastrointestinal tract and thereby provide a sustained action over a longer period. For example, a time delay such as glyceryl monostearate or glyceryl distearate alone or with a wax can be employed.

Formulations for oral use can also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

Aqueous suspensions of the invention contain the active materials in admixture with excipients suitable for the manufacture of aqueous suspensions. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (*e.g.*, lecithin), a condensation product of an alkylene oxide with a fatty acid (*e.g.*, polyoxyethylene stearate), a condensation product of ethylene oxide with a long chain aliphatic alcohol (*e.g.*, heptadecaethylene oxycetanol), a condensation product of ethylene oxide with a partial ester derived from a fatty acid and a hexitol (*e.g.*, polyoxyethylene sorbitol mono-oleate), or a condensation product of ethylene oxide with a partial ester derived from fatty acid and a hexitol anhydride (*e.g.*, polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Ophthalmic formulations, as is known in the art, will be adjusted for osmolarity.

Oil suspensions can be formulated by suspending the active ingredient in a vegetable oil, such as arachis oil, olive oil, sesame oil or coconut oil, or in a mineral oil such as liquid paraffin. The oil suspensions can contain a thickening agent, such as beeswax, hard paraffin or cetyl alcohol. Sweetening agents can be added to provide a palatable oral preparation. These compositions can be preserved by the addition of an antioxidant such as ascorbic acid.

Dispersible powders and granules of the invention suitable for preparation of an aqueous suspension by the addition of water can be formulated from the active ingredients in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, can also be present.

The pharmaceutical compositions of the invention can also be in the form of oil-in-water emulsions. The oily phase can be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion can also contain sweetening and flavoring agents.

Syrups and elixirs can be formulated with sweetening agents, such as glycerol, sorbitol or sucrose. Such formulations can also contain a demulcent, a preservative, a flavoring or a coloring agent.

The pharmaceutical compositions of the invention can be in the form of a sterile injectable preparation, such as a sterile injectable aqueous or oleaginous suspension. This suspension can be formulated according to the known art using those suitable dispersing or wetting agents and suspending agents which have been mentioned above. The sterile injectable preparation can also be a sterile injectable solution or suspension in a nontoxic parenterally-acceptable diluent or solvent, such as a solution of 1,3-butanediol. Among the acceptable vehicles and solvents that can be employed are water and Ringer's solution, an isotonic sodium chloride. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

The compounds of this invention can also be administered in the form of suppositories for rectal administration of the drug. These compositions can be prepared by mixing the drug with a suitable non-irritating excipient which is solid at ordinary temperatures but liquid at the rectal temperatures and will therefore melt in the rectum to release the drug. Such materials are cocoa butter and polyethylene glycols.

They can also be administered by in intranasal, intraocular, intravaginal, and intrarectal routes including suppositories, insufflation, powders and aerosol formulations.

Products of the invention which are preferably administered by the topical route can be administered as applicator sticks, solutions, suspensions, emulsions, gels, creams, ointments, pastes, jellies, paints, powders, and aerosols.

## EXAMPLE 1

This example illustrates the preparation and properties of 17α,21-Dimethoxy-11β-[4-(N,N-dimethylamino)phenyl]-19-norpregna-4,9-diene-3,20-dione (**113a**) (**Figure 8**):

### *Step 1. 3,3-Eltlyleizedioxy-17α-metlioxy-21-hydroxy-19-norpregna-5(10),9(11)-dien-20-one (107):*

To a solution of the 17α-methoxy-3-ketal (94, 10.0 g, 27.1 mmol) in dry THF (150 mL) was added iodobenzene diacetate (Moriarty, et al., J. Chem. Soc., Chem. Commun., 641-642 (1981); Velerio, et al., Steroids, 60:268-271 (1995) ) (34.59 g, 4x) as a solid. The suspension was stirred under nitrogen and cooled to 0°C. $H_2O$ (7.73 mL, 429.6 mmol, 16x) was added, followed by 0.5 M KO-tBu solution (1400 mL, 700 mmol, 26x) *via* transfer needle. (A 50:50 (v/v) mixture of freshly opened methanol (700 mL) and 1.0 M potassium t-butoxide in THF (700 mL; Aldrich) was prepared and cooled to 0°C to give a 0.5 M base solution). Upon completion of addition the reaction mixture was removed from the ice bath and the solution alowed to warm to room temperature. The reaction was monitored every hour by TLC (5% acetone in $CH_2Cl_2$) and after 4 hr, virtually all of the starting material had been converted to approximately a 80:20 mixture of two more polar components. The reaction mixture was diluted with $H_2O$ (500 mL) and brine (500 mL) and extracted into ether (3x). Organic fractions were washed again with $H_2O$ and brine. Combined organic extracts were dried by filtration through $Na_2SO_4$, evaporated in *vacuo,* and further dried under high vacuum to recover 13.84 g of an orange oil. Purification by flash chromatography (5% acetone in $CH_2Cl_2$) gave 6.0 g of a pale yellow-white foam (**107**) in 57.5% yield. Trituration with pentane produced **107** which was dried under vacuum to recover 5.36 g of a white powder in 51.0% yield; m.p. = 147 - 152°C. FTIR (KBr, diffuse reflectance): $V_{max}$ 3478, 2900, 2825, 1712, 1437, 1384 and 1372 $cm^{-1}$. NMR (300 MHz, $CDCl_3$): δ 0.550 (s, 3 H, C18-$CH_3$), 3.159 (s, 3 H, C17α-$OCH_3$), 3.981 (s, 4 H, C3-$OCH_2CH_2O$), 4.251 and 4.471 (AB,

2 H, $J_{AB}$ = 19.81 Hz, C21-$CH_2$) and 5.544 (br s, 1 H, C11-CH=). MS (EI) m/z (relative intensity): 388 ($M^+$, 54.8), 356 (13.8), 297 (100.0), 211 (65.0), 169 (51.1) and 99 (56.3). Anal. Calcd. for $C_{23}H_{32}O_5 \cdot 1/4H_2O$: C, 70.29; H, 8.34. Found: C, 70.21; H, 8.12.

***Step 2. 3,3-Ethylenedioxy-17α,21-dimethoxy-19-norpregna-5(10),9(11)-dien-20-one (108):***

To a solution of the 3-ketal-21-hydroxy compound (107, 5.0 g, 12.87 mmol) in 500 mL of 1,2-dimethoxyethane (DME) was added Proton-Sponge® [1,8-bis(dimethylaniino)naphthalene] (13.79 g, 64.35 mmol, 5x) as a solid. The solution was cooled to 0°C in an ice water bath and trimethyloxonium tetrafluoroborate (9.52 g, 64.35 mmol, 5x) was added as a solid. The suspension was kept at 0°C under nitrogen, for 3 hr. At that time, TLC (5% acetone in $CH_2Cl_2$) indicated all of the starting material had been cleanly converted to the slightly less polar 3-ketal-17α,21-dimethoxy compound (**108**). $H_2O$ and EtOAc were added, the mixture was transferred to a separatory funnel, and the layers allowed to separate. The organic fraction was washed with ice-cold 1 N HCl (2x), $H_2O$ (1x), saturated $NaHCO_3$ (1x), $H_2O$ (1x), and brine (1x). Combind EtOAc extracts (3x) were dried by filtration through $Na_2SO_4$ and evaporated *in vacuo.* The resulting colorless oil was dried overnight under high vacuum to recover a white foam (**108,** 5.28 g) in quantitative yield. Analysis by TLC and NMR indicated the crude material was sufficiently pure to carry directly on to the next reaction. A small amount was triturated with pentane and dried overnight under high vacuum to give 120 mg of **108** as a white solid; m.p = 104 - 110°C. FTIR (KBr, diffuse reflectance): $V_{max}$ 2926, 2884, 2828, 1722, 1447, 1380, 1322 and 1252 $cm^{-1}$. NMR (300 MHz, $CDCl_3$): δ 0.585 (s, 3 H, C18-$CH_3$), 3.175 (s, 3 H, C17α-$OCH_3$), 3.442 (s, 3 H, C21-$OCH_3$), 3.983 (s, 4 H, C3-O$CH_2CH_2$O), 4.182 and 4.367 (AB, 2 H, $J_{AB}$ = 18.01 Hz, C21-$CH_2$) and 5.555 (br s, 1 H, C11-CH=). MS (EI) m/z (relative intensity): 402 ($M^+$, 27.7), 370 (7.2), 297 (100.0), 211 (62.1), 169 (41.6) and 99 (62.7). Anal. Calcd. for $C_{24}H_{34}O_5 \cdot 3/5H_2O$: C, 69.74; H, 8.58. Found: C, 69.82; H, 8.43.

***Step 3. 3,3 Ethylenedioxy-17α,21-dimethoxy-19-norpregna-5(10),9(11)-dien-20-ol (109):***

The 3-ketal 17α,21-dimethoxy-20-one (**108**, 5.0 g, 12.42 mmol) was dissolved in dry THF (100 mL) and 2 equivalents of $LiAlH_4$ (25 mL, 25 mmol, 1.0 M in ether) were added *via* syringe. The solution was stirred magnetically at room temperature under nitrogen. After 15 minutes, examination by TLC (5% acetone in $CH_2Cl_2$) indicated the starting material had been cleanly converted to a single, more polar product (**109**). The reaction mixture was cooled in an ice bath, and saturated $Na_2SO_4$ (~2 - 3 mL) was added dropwise *via* pipette. When the reaction was quenched, several scoops of $Na_2SO_4$ were added and the mixture allowed to stir 1 hr. Filtration through a sintered glass funnel, followed by evaporation in *vacuo* produced a concentrated syrup. The syrup was taken up in $H_2O$ and $CH_2Cl_2$, transferred to a separatory funnel, and the layers allowed to separate. The organic fraction was washed again with brine. Combined $CH_2Cl_2$ extracts (3x) were dried by filtration through $Na_2SO_4$ and evaporated *in vacuo.* The resulting white foam was dried further under high vacuum to recover 4.69 g of the crude **109**. Purification of this crude product by flash chromatography (5% isopropanol in $CH_2Cl_2$) gave 4.24 g of **109** as a white foam in 84.4% yield.

The two purest fractions were combined and taken up in a minimum amount of acetone/hexane. After standing six days at room temperature, large, colorless crystals had formed. The crystals were collected by centrifugation, washed with several portions of hexane, and dried under high vacuum to recover 177 mg. Analysis by TLC (10% acetone in $CH_2Cl_2$) indicated the crystals were of the highest purity. Analysis of this material by NMR indicated a single isomer. No further work was done for identification of this single isomer. A second crop of 78 mg with only a trace of impurity was obtained from the mother liquors; m.p. =111 - 115°C. FTIR (KBr, diffuse reflectance): $v_{max}$ 3576, 3456, 2930, 2891, 2827, 1460 and 1372 $cm^{-1}$. NMR (300 MHz, $CDCl_3$): δ 0.824 (s, 3 H, C18-$CH_3$), 3.298 (s, 3 H, C17α-$OCH_3$), 3.392 (s, 3 H, C21-$OCH_3$), 3.416 (dd, 1 H, $J_1$ = 9.30 Hz, $J_2$ = 8.10 Hz, C21-$CH_2$), 3,490 (dd, 1 H, $J_1$ = 9.30 Hz, $J_2$ = 3.30 Hz, C21-$CH_2$), 3.923 (dd, 1 H, $J_1$ = 8.10 Hz, $J_2$ = 3.30 Hz, C20-CH), 3.980 (s, 4 H, C3-O$CH_2CH_2$O) and 5.595 (br s, 1 H, C11-CH=). MS (EI) m/z (relative intensity): 404 ($M^+$, 2.1), 372 (5.7), 329 (1.7), 297 (100.0) and 211 (35.7). Anal. Calcd. for $C_{24}H_{36}O_5 \cdot 1/5C_6H_{14}$: C, 71.76; H, 9.27. Found: C, 71.83; H, 9.04.

***Step 4. 3,3-Ethylenedioxy-5a,10a-epoxy-17a,21-dimethxy-19-norpregn-9(11)-en-20-ol (110):***

To a solution of hexafluoroacetone (2.01 mL, 14.39 mmol) in $CH_2Cl_2$ (50 mL), was added solid $Na_2HPO_4$ (1.36 g, 9.59 mmol) and 30% $H_2O_2$ (2.16 mL, 21.1 mmol). The mixture was transferred to the cold room and stirred vigorously for ½ hr at 4°C. A chilled solution of the 20-alcohol (**109**, 3.88 g. 9.59 mmol) in $CH_2Cl_2$ (25 mL) was added via pipette and rinsed in with additional CH $2Cl_2$ (25 mL). After stirring overnight at 4°C, TLC (7.5% acetone in $CH_2Cl_2$) indicated virtually all of the starting material had been converted to one major, more polar product with only a trace of byproducts. The reaction mixture was transferred to a separatory funnel and washed with 10% $Na_2SO_3$ (1x), $H_2O$ (1x), and brine (1x). Combined $CH_2Cl_2$ extracts (3x) were dried by filtration through $Na_2SO_4$ and evaporated in *vacuo* to recover a foam. NMR analysis of the crude material indicated the α and β epoxides were present in approximately a 9:1 ratio. Trituration

with ether produced 2.27 g of the pure 5α,10 α epoxide (**110**) as a white powder in 56.3% yield; m.p. =146 - 153°C. FTIR (KBr, diffuse reflectance): $V_{max}$ 3558, 2939, 1638, 1446, 1373 and 1247 $cm^{-1}$. NMR (300 MHz, $CDCl_3$): δ 0.824 (s, 3 H, C18-$CH_3$), 3.273 (s, 3 H, C17α-$OCH_3$), 3.389 (s, 3 H, C21-$OCH_3$), 3.402 (dd, 1 H, $J_1$ = 9.61 Hz, $J_2$ = 8.10 Hz, C21-$CH_2$), 3,476 (dd, 1 H,$J_1$= 9.1 Hz, $J_2$ = 3.30 Hz, C21-$CH_2$), 3.908 (m, 5 H, C3-O$CH_2CH_2$O and C20-CH) and 6.053 (br s, 1 H, C11-CH=). MS (EI) m/z (relative intensity): 420 ($M^+$, 1.7), 402 (6.0), 370 (6.2), 345 (20.0), 313 (77.8), 295 (100.0) and 99 (95.4). Anal. Calcd. for $C_{24}H_{36}O_5$-1/10$H_2O$: C, 68.25; H, 8.64. Found: C, 68.31; H, 8.71.

***Step 5. 3,3-Ethylenedioxy-5α-hydroxy-11β-[4-(N,N-dimethylamino)phenyl]-17α,21-dimethoxy-19-norpregn-9-en-20-ol (111a):***

A dry 50 mL 2-neck flask was equipped with a stirrer, a reflux condenser, and a rubber septum. Magnesium (191 mg, 7.85 mmol) was added and the entire apparatus was dried fruther, under a stream of nitrogen, with a heat gun. After cooling slightly, one crystal of iodine was added. The apparatus was allowed to cool completely and dry THF (4 mL) was added followed by one drop of 1,2-dibromoethane. A solution of 4-bromo-N,N-dimethylaniline (1.43 g, 7.14 mmol) in THF (2 mL) was added *via* transfer needle and rinsed in with additional THF (2.0 mL). The mixture was warmed gently with a heat gun to initiate reaction (as evidenced by bleaching of color) and then allowed to stir 1 hr at ambient temperature. Copper (I) chloride (78.2 mg, 0.79 mmol) was added as a solid and stirring continued for 20 min. A solution of the 5α, 10α-epoxide (**110**, 1.0 g, 2.38 mmol) in THF (4.0 mL, heated gently to achieve a solution) was added *via* transfer needle and rinsed in with additional THF (2 x 2.0 mL). After stirring 2 hr at room temperature, the reaction was quenched by the addition of saturated $NH_4Cl$ (16 mL). Air was drawn through the mixture for ½ hr with vigorous stirring. The mixture was transferred to a separatory funnel, ether was added, and the layers allowed to separate. The organic fraction was washed again with $H_2O$ (1x), and brine (1x). Combined ether extracts (3x) were dried by filtration through $Na_2SO_4$ and evaporated *in vacuo* to recover an oily residue. Trituration with ether produced a solid **111a.** The crystals were collected on a Buchner funnel, triturated with additional ether, and dried under high vacuum to recover 1.02 g of a beige solid (**111a**) in 79% yield; m.p. =195-199°C. FTIR (KBr, diffuse reflectance): $v_{max}$ 3534, 3418, 2938, 2875, 2820, 1868, 1614, 1560, 1519, 1443, 1353 and 1328 $cm^{-1}$. NMR (300 MHz, $CDCl_3$): δ 0.493 (s, 3 H, C18-$CH_3$), 2.896 (s, 6 H, -N$(CH_3)_2$), 3.289 (s, 3 H, C17α-$OCH_3$), 3.362 (s, 3 H, C21-$OCH_3$), 3.340 - 3.448 (m, 2 H, C21-$CH_2$), 3.747 - 4.075 (m, 5 H, C3-O$CH_2CH_2$O and C20-CH), 4.171 (br s, 1 H, C 11α-CH), 6.63 5 (d, 2 H, J = 8.70 Hz, 3', 5' aromatic-CH's) and 7.070 (d, 2 H, J = 8.70 Hz, 2', 6' aromatic-CH's). MS (E1) m/z (relative intensity): 541 ($M^+$, 61.0), 523 (19.7), 416 (7.6), 134 (37.4), 121 (100.0) and 99 (20.2). Anal. Calcd. for $C_{32}H_{47}NO_6$: C, 70.95; H, 8.74; N, 2.59. Found: C, 70.92; H, 8.77; N, 2.65.

***Step 6. 3,3-Ethylenedioxy-5α-hydroxy-11β-[4-(N,N-dimethylamino)phenyl]-17α,21-dimethoxy-19-norpregn-9 (10)-en-20-one (112a):***

**(a) Preparation of o-iodoxybenzoic acid** (Dess, et al., J Org. Chem., 48:4155-4156 (1983)): The initial preparation of IBX gave a material which appeared to be a mixture as evidenced by $^{13}C$ NMR. Although the oxidant was not homogenous, 3 equivalents of this material (assuming 100% IBX) cleanly converted the 20-OH (**111a**) to the 20-ketone (**112a**). The preparation of IBX has been since modified to obtain a homogeneous material with $^1H$ NMR and $^{13}C$ NMR identical to the reported spectra (Frigerio, et al., Tet. Letters, 35:8019-8022 (1994)). Only 1.5 equivalents are necessary for oxidation (Frigerio, et al., Tet. Letters, 35:8019-8022 (1994); Frigerio, et al., J. Org. Chem., 60:7272-7276 (1995)). This new material was used for the preparation of **112b** and **112c.**

Potassium bromate (7.6 g, 45.5 mmol) was added over a 10 minute period to a vigorously stirred suspension of 2-iodobenzoic acid (8.52 g, 34.4 mmol) in 0.73 M $H_2SO_4$ (150 mL). Upon completion of addition, the mixture was warmed to 65°C in a water bath. Over the next hour, bromine was evolved as was evidenced by a change in color from orange to white. At that time, a second aliquot of potassium bromate (7.6 g, 45.5 mmol) was added and stirring continued at 65°C for an additional 2 hr. The mixture was cooled to room temperature, filtered on a Buchner funnel, and washed with $H_2O$, followed by acetone. The resulting white solid was dried in vacuo to recover 7.74 g in 80.2% yield. $^1H$ NMR (300 MHz, DMSO): δ 7.845 (t, 1 H, J = 7.20 Hz), 7.96 - 8.06 (m, 2 H) and 8.148 (d, 1 H, J = 7.80 Hz). $^{13}C$ NMR (300 MHz, DMSO): δ 125.011, 130.093, 131.398, 132.963, 133.406, 146.525 and 167.499.

**(b) Oxidation of the 20-ol (111a) to the 20-one (112a):** To a solution of IBX (2.42 g, 8.64 mmol) in DMSO (16.0 mL) at ambient temperature, under nitrogen, a solution of the Grignard product (**111a,** 1.56 g, 2.88 mmol) in DMSO (16.0 mL) was added *via* transfer needle. Additional DMSO (2 x 4.0 mL) was used to rinse in residual steroid. The resulting purple solution was stirred ½ hr. At that time, examination by TLC (10% acetone in $CH_2Cl_2$; aliquot was diluted in $H_2O$ and extracted into EtOAc) revealed all of the starting material had been cleanly converted to a single, less polar product. The reaction was transferred to a separatory funnel, $H_2O$ and $CH_2Cl_2$ were added, and the layers

allowed to separate. The organic fractions were washed again with $H_2O$ (1x) and then brine (1x). Combined $CH_2Cl_2$ exracts (3x) were dried by filtration through $Na_2SO_4$ and evaporated *in vacuo.* The resulting residue was dried overnight under high vacuum to recover a brownish-purple gum (1.79 g). The gum was taken up in $CH_2Cl_2$ and filtered through silica (~250 mL) on a sintered glass funnel. After eluting with $CH_2Cl_2$ to remove DMSO (2 x 250 mL), the pure product was eluted with 10% acetone in $CH_2Cl_2$ (2 x 250 mL). Fractions containing the product were combined, evaporated *in vacuo* and dried briefly under high vacuum to afford 1.29 g of **112a** as a colorless foam in 83% yield: A small sample (~100 mg) was reserved, triturated with pentane, and dried to give a white crystalline solid; m.p. =160 -165°C. FTIR (KBr, diffuse reflectance): $\nu_{max}$ 3514, 2938, 2824, 1724, 1616, 1521, 1520, 1447 and 1354 $cm^{-1}$. NMR (300 MHz, $CDCl_3$): $\delta$ 0.250 (s, 3 H, C18-$CH_3$), 2.894 (s, 6 H, -$N(CH_3)_2$), 3.137 (s, 3 H, C17α-$OCH_3$), 3.435 (s, 3 H, C21-$OCH_3$), 3.998 (m, 4 H, C3-$O\underline{CH_2CH_2}O$), 4.231 and 4.363 (AB, 2 H, $J_{AB}$ =18.01 Hz, C21-$CH_2$), 4.250 (br d, I H, C11α-CH), 4.288 (br s, 1 H, C5α-OH), 6.619 (d, 2 H, *J* = 8.85 Hz, 3', 5' aromatic-CH's) and 7.016 (d, 2 H, J = 8.85 Hz, 2', 6' aromatic-CH's). MS (EI) m/z (relative intensity): 539 ($M^+$, 71.4), 521 (34.8), 134 (52.9), 121 (100.0) and 99 (23.5). Anal. Calcd. for $C_{32}H_{45}NO_6$: C, 71.21; H, 840; N, 2.60. Found: C, 71.41; H, 8.60; N, 2.63.

***Step 7. Preparation of the target compound 113a:***

To a solution of the 3-ketal-Sa-hydroxy-20-one (**112a**, 1.20 gm 2.22 mmol) in THF (15.0 mL), was added glacial acetic acid (45.0 mL, 783 mmol), followed by $H_2O$ (15.0 mL). The mixture was brought to reflux under nitrogen. After 1 hr., TLC (25% EtOAc in $CH_2Cl_2$) indicated the 3-ketal had been hydrolyzed to give the slightly less polar ketone. The reaction was allowed to cool to room temperature and left overnight under nitrogen. Concentrated $NH_4OH$ (53.0 mL, 783 mmol) was added to neutralize the reaction and additional $NH_4OH$ was added to bring the mixture to pH 7.0 (paper). The mixture was transferred to a separatory funnel and extracted into $CH_2Cl_2$ (3x). The organic fractions were washed again with $H_2O$ (1x) and brine (1x). Combined $CH_2Cl_2$ extracts were dried by filtration through $Na_2SO_4$ and evaporated *in vacuo* to give 1.21 g of a yellow oil. The crude product was purified twice by flash chromatography (7.5% acetone in $CH_2Cl_2$). Fractions containing the pure product were combined and evaporated to give a yellow gum. Trituration with heptane produced 350 mg of a pale yellow powder. All remaining material (impure fractions plus mother liquors) was combined and rechromatographed to give an additional 305 mg: Total yield was 655 mg of **113a** in 61.7% yield; m.p. =132 - 136°C. HPLC analysis of **113a** on a Waters Assoc. NovaPak $C_{18}$ column eluted with 30% 50 m$\underline{M}$ $KH_2PO_4$ (pH = 3.0) in MeOH at a flow rate of 1 mL per min and at $\lambda$ = 302 nm indicated a purity of 97.9% with a retention time ($t_R$) of 7.87 min. FTIR (KBr, diffuse reflectance): $\nu_{max}$ 2946, 1724, 1665, 1599, 1518, 1445 and 1348 $cm^{-1}$. NMR (300 MHz, $CDCl_3$): $\delta$ 0.322 (s, 3 H, C18-$CH_3$), 2.904 (s, 6 H, -$N(CH_3)_2$), 3.173 (s, 3 H, C17α-$OCH_3$), 3.453 (s, 3 H, C21-$OCH_3$), 4.234 and 4.375 (AB, 2H, $J_{AB}$ =17.86 Hz, C21-$CH_2$), 4.367 (s, 1 H, C11α-CH), 5.750 (s, 1 H, C4-CH=), 6.634 (d, 2H, J = 8.55 Hz, 3', 5' aromatic-CH's) and 6.979 (d, 2 H, J = 8.55 Hz, 2', 6' aromatic-CH's). MS (EI) m/z (relative intensity): 477 ($M^+$, 83.2), 372 (10.3), 251 (17.1),209 (20.4), 134 (35.3) and 121 (100.0). Anal. Calcd. for $C_{30}H_{39}NO_4$: C, 75.44; H, 8.23; N, 2.93. Found: C, 75.54; H, 8.14; N, 2.94.

**EXAMPLE 2**

This example illustrates the preparation and properties of 17α-Acetoxy-11β-[4-(N,N-dimethylamino)phenyl]-21-(2'-methoxyacetyl)oxy-19-norpregna-4,9-diene-3,20-dione (**126b**) (**Figure 11**):

***Step 1. 17α-Hydroxy-11β-(4-(N,N dimethylan:ino)phenyl]-21-(2'-methoxyacetyl)oxy-19-norpregna-4,9-diene-3,20-dione (125b):***

Under nitrogen, a solution of the 17α,21-diol (**124**, 1.0 g, 2.22 mmol), pyridine (1 mL, 12.41 mmol) and triethylamine (1 mL, 7.11 mmol) in dry benzene (40 mL) was treated with methoxyacetyl chloride (0.5 mL, 5.47 mmol). The reaction mixture was stirred at room temperature for 4 hr, after which time TLC (5% isopropanol in $CH_2Cl_2$) indicated a complete reaction. Solvents were removed *in vacuo* under a current of nitrogen and the residue was diluted with $H_2O$ (~50 mL) and extracted with $CH_2Cl_2$ (3x). The organic fractions were washed with $H_2O$ (3x), filtered .through anhydrous $Na_2SO_4$, combined and concentrated *in vacuo* to give 1.4 g of the residue as a yellow solid. This material was purified by flash chromatography (3% isopropanol in $CH_2Cl_2$) to give 1.05 g of the product as a yellow foam. Crystallization from ether containing a small amount of $CH_2Cl_2$ gave 0.73 g of the pure 21-(2'-methoxy)-acetyloxy-derivative **125b** as an off-white solid in 62.9% yield; m.p. = 197 - 199°C. FTIR (KBr, diffuse reflectance): $V_{max}$ 3329, 2948, 2888, 1754, 1729, 1637, 1602 and 1518 $cm^{-1}$. NMR (300 MHz, $CDCl_3$): $\delta$ 0.399 (s, 3 H, C18-$CH_3$), 2.906 (s, 6 H, -$N(CH_3)_2$), 3.488 (s, 3 H, C21-$OCH_3$), 4.181 (s, 2 H, C21-OC(O)$\underline{CH_2}$-), 4.384 (d, 1 H, J = 4.384, C11α-CH), 4.975 and 5.234 (both d, 2 H, J = 17.4 Hz, C21-$CH_2$), 5.760 (s, 1 H, C4-CH=), 6.654 (d, 2 H, J = 8.7 Hz, 3', 5' aromatic-CH's) and 7.012 (d, 2 H, J = 8.7 Hz, 2', 6' aromatic-CH's). MS(EI) m/z (relative intensity): 521 ($M^+$, 26.4), 431 (7.1), 134 (17.3) and 121 (100.0). Anal.

Calcd. for $C_{31}H_{39}NO_3$: C, 71.38; H, 7.54; N, 2.69. Found: C, 71.48; H, 7.59; N, 2.64.

***Step 2. Preparation of the target compound 126b:***

Under nitrogen, trifluoroacetic anhydride (2.98 g, 14.16 mmol), glacial acetic acid (0.84 g, 13.98 mmol) and dry $CH_2Cl_2$ (5 mL) were combined and stirred at room temperature for ½ hr. Toluenesulfonic acid monohydrate (0.15 g, 0.79 mmol) was added and the mixture cooled to 0°C in an ice bath. A solution of the 21-(2'-methoxy)acetyloxy-17α-ol (**125b**, 0.612 g, 1.173 mmol) in dry $CH_2Cl_2$ (2 mL) was added and the reaction was stirred at 0°C and monitored by TLC (3% isopropanol in $CH_2Cl_2$) which indicated a complete reaction after 4 hr. The mixture was diluted with $H_2O$ (~10 mL), stirred at 0°C for another 15 minutes, then carefully neutralized with dropwise addition of concentrated $NH_4OH$ solution (~3 mL). The mixture was extracted with $CH_2Cl_2$ (3x). The organic fractions were washed with $H_2O$ (2x) and brine (1x), filtered through anhydrous $Na_2SO_4$, combined and concentrated *in vacuo* to give 0.72 g of the residue as an oil. This material was purified *via* flash chromatography (20% EtOAc in $CH_2Cl_2$) to give 0.34 g of **126b** as a yellow foam. Trituration of this material with pentane gave 0.26 g of the pure title compound (**126b**) as a light yellow amorphous solid in 39.3% yield; m.p. =110 -113°C.

Analysis of 126b by HPLC on a Waters NovaPak, $C_{18}$ column, eluted with 0.05 M KH $_2$PO $_4$ buffer [pH = 3.0] /MeOH, 35:65 at a flow rate of 1 mL per min and at λ = 302 nm indicated this material to be >99% pure with a retention time ($t_R$ = 6.04 min). FTIR (KBr, diffuse reflectance): $v_{max}$ 2947, 1766, 1737, 1663, 1612 and 1518 $cm^{-1}$. NMR (300 MHz, $CDCl_3$): δ 0.447 (s, 3 H, C18-$CH_3$), 2.129, (s, 3 H, C17α-OAc), 2.907 (s, 6 H, - N($CH_3$)$_2$), 3.473 (s, 3 H,C21-OC (O)$CH_2$O$CH_3$), 4.176 (s, 2 H, C21-OC(O)$CH_2$-), 4.392 (d, 1 H, J = 6 Hz, C11α-CH), 4.792 and 5.029 (both d, 2 H, J = 17.4 Hz, C21-$CH_2$), 5.777 (s, 1 H, C4-CH=), 6.644 (d, 2 H, J = 9 Hz, 3', 5' aromatic-CH's) and 7.002 (d, 2 H, J = 9 Hz, 2', 6'-aromatic-CH's). MS (EI) m/z (relative intensity): 563 ($M^+$, 42.8), 503 (12.6), 134 (17.2) and 121 (100.0). Anal. Calcd. for $C_{33}H_{41}NO_7$: C, 70.32; H, 7.33; N, 2.48. Found: C, 70.14; H, 7.59; N, 2.41.

**EXAMPLE 3**

This example illustrates the preparation and properties of 17α-Acetoxy-11β-[4-(N,N-dimethylamino)phenyl]-21-(1'-ethenyloxy)-19-norpregna-4,9-diene-3,20-dione (**129**) (**Figure 11**):

***Step 1. 17α,21-(1'-Ethoxyethylidenedioxy)-11β-[4-(N,N-dimethylamino)phenyl]-19-norpregna-4,9-diene-3,20-dione (127):***

The 17α,21-diol (10) (**124**, 1.6 g, 3.56 mmol), triethyl orthoacetate (5.59 g, 3.45 mmol), and pyridinium tosylate (200 mg, 0.93 mmol) were dissolved in dry benzene in a nitrogen atmosphere and heated at reflux for 75 min using a Dean Stark trap to remove water. The reaction was complete at this time. Pyridine (1 mL) was added and the solvent was evaporated using nitrogen and vacuum. Water was added and the mixture was extracted with $CH_2Cl_2$ (3x). The $CH_2Cl_2$ extracts were washed with $H_2O$, brine and dried over anhydrous $Na_2SO_4$. The solvent was evaporated in *vacuo.* Purification by dry column chromatography, recrystallization and finally flash column chromatography using $CH_2Cl_2$: acetone (97:3) gave 1.028 g of the ortho ester (**127**) in 55.8% yield. NMR ($CDCl_3$): δ 0.334 (s, 3 H, C18-$CH_3$), 1.620 (s, 3 H, C17α,2-ethylidenedioxy-$CH_3$), 2.909 (s, 6 H, N($CH_3$)$_2$), 3.55 (q, 2 H, C21-ethylidendioxy-O$CH_2$$CH_3$), 4.404 (br d, 1 H, C11α-CH), 5.769 (s, 1 H, C4-CH=), 6.641 (d, 2 H, 3', 5' aromatic-CH's) and 7.003 (d, 2 H, 2', 6' aromatic-CH's).

***Step 2. 17α-Acetoxy-11β-[4-(N,N-dimethylamino)phenyl]-21-hydroxy-19-norpregna-4,9-diene-3,20-dione (128):***

The cyclic ortho ester (**127**, 1.028 g, 1.99 mmol) was suspended in methanol (60 mL) in a nitrogen atmosphere and NaOAc solution (8.2 mL, 0.1 M) and HOAc solution (16.4 mL, 0.2 M) were added. The mixture was heated at reflux for 3 hr. The solvent was evaporated using nitrogen and vacuum. $H_2O$ (~50 mL) was added and the mixture was extracted wtih $CH_2Cl_2$ (3x). The organic fractions were washed with $H_2O$, brine and dried over anhydrous $Na_2SO_4$ to give 1.0112 g of the 17 α-acetoxy-21-hydroxy compound (**128**) as an off-white powder containing a trace amount of the 17α-hydroxy-11β-[4-(N,N-dimethylamino)phenyl]-21-acetoxy-19-norpregna-4,9-diene-3,20-dione compound (**8**). The crude product was chromatographed on flash column silica gel using $CH_2Cl_2$:acetone (8:2) as the solvent. Fractions were collected and each fraction was checked by TLC. Fractions #5 - 7 were essentially pure **128** and were combined to give 108.5 mg of good product. The residue was crystallized from ether to give 75 mg of an additional pure **128**. The total amount of the product **128** was 183.5 mg as an off- white powder in 18.8% yield; m.p. = 205 -210°C. NMR ($CDCl_3$): δ 0.364 (s, 3 H, C18-$CH_3$), 2.112 (s, 3 H, C17α-OAc), 2.902 (s, 6 H, -N($CH_3$)$_2$), 4.190 - 4.405 (br d and m, 3 H, C11α-CH and C21-$CH_2$-), 5.779 (br s, 1H, C4-CH=), 6.629 (d, 2 H, 3', 5' aromatic-CH's) and 6.967 (d, 2 H, 2', 6' aromatic-CH's).

***Step 3. Preparation of the target compound 129:***

The 21-hydroxy compound (**128**, 682 mg, 1.39 mmol) was dissolved in $CH_2Cl_2$ (14 mL) in a nitrogen atmosphere and ethyl vinyl ether (5.27 g, 7.32 mmol) was added. Mercury (II) trifluoroacetate (25 mg, 0.059 mmol) was added and the mixture was stirred in a nitrogen atmosphere at room temperature for 22 hr. The mixture was poured onto dry column silica gel which had been washed with $CH_2Cl_2$ in a sintered glass funnel. The compound was eluted with EtOAc and the solvent was evaporated *in vacuo.* The residue (744 mg) was chromatographed on Flash column silica gel using $CH_2Cl_2$: acetone (95:5) as the solvent. A total of 141 mg of good product **129** was obtained as a yellow foam in 19.6% yield. The compound **129** was dried to remove ether; m.p. = 114 - 116°C. Analysis of **129** by HPLC on a NovaPak $C_{18}$ column eluted with MeOH:$H_2O$:$Et_3N$ (70:30:0.05) at a flow rate of 1 mL per min and at $\lambda$ = 260 nm indicated it to be better than 99% pure. FTIR (KBr, diffuse reflectance): $\nu_{max}$ 2948, 1733, 1662, 1613, 1560, 1518, 1446, 1369, 1278 and 1235 $cm^{-1}$. NMR ($CDCl_3$): $\delta$ 0.408 (s, 3 H, C18-$CH_3$),2.118 (s, 3 H, C17$\alpha$-OAc), 2.901 (s, 6 H, -N($CH_3$)$_2$), 4.096 - 4.662 (m, 6 H, C21-Ovinyl H, C11$\alpha$-CH and C21-$CH_2$-), 5.779 (br s, 1 H, C4-CH=), 6.625 (d, 2 H, 3', 5' aromatic-CH's), and 6.967 (d, 2 H, 2', 6' aromatic-CH's). MS (EI) m/z (relative intensity): 517($M^+$, 73), 134 (18.0) and 121 (100.0). Anal. Calcd. for $C_{32}H_{39}NO_6 \cdot 1/3H_2O$: C, 73.40; H, 7.64; N, 2.67. Found: C, 73.49; H, 7.62; N, 2.84.

**REFERENCE EXAMPLE**

This example illustrates the preparation of compound 94, which is used in the preparation of compound 113a. See Figure 6.

***Step 1. 17α-Hydroxy-19-norpregna-9,9-diene-3,20-dione <u>(92)</u>:***

Under nitrogen, the diketal (**50**, 20.0 g, 49.7 mmol) was dissolved in a mixture of THF (333 mL) and $H_2O$ (333 mL) followed by trifluoroacetic acid (1 L, 13.46 mmol). The reaction mixture was then stirred at room temperature for 2 hr, after which time, TLC (10% acetone in $CH_2Cl_2$, overspotted with concentrated $NH_4OH$ indicated a complete reaction. The reaction mixture was cooled in an ice bath and neutralized by the dropwise addition of concentrated (29.5%) $NH_4OH$ (862 mL, ~13.46 mol) over a period of about an hour. The reaction mixture was diluted with $H_2O$ (~500 m L) and extracted with methylene chloride (3x). The organic fractions were washed with saturated $NaHCO_3$ (1x) and $H_2O$ (1x), brine (1x), then filtered through anhydrous sodium sulfate, combined and concentrated *in vacuo.* Crystallization of the residue from acetone/hexanes gave 12 g of the pure product **92** as a white crystalline solid in 76.8% yield; m.p. = 203-205°C. FTIR (KBr, diffusion reflectance) $\nu_{max}$ 3438, 2950, 1702, 1642 and 1593 $cm^{-1}$. $^1H$ NMR (300 MHz, $CDCl_3$) $\delta$ 0.857 (s, 3 H, C18-$CH_3$), 2.289 (s, 3 H, C21-$CH_3$) and 5.669 (s, 1 H, C4-CH=). $^{13}C$ NMR ($CDCl_3$): $\delta$ 14.703, 23.901, 25.341, 25.714, 27.515, 27.615, 30.260, 30.765, 33.470, 36.971, 39.086, 47.846, 50.696. 89.565 (C17), 122.015 (C4), 125.440(C10). 145.632 (C9). 157.339 (C5), 199.824 (C3) and 211.201 (C20). MS (EI) m/z (relative intensity): 314 ($M^+$, 100), 296 (13.6), 271 (58.0), 213 (67.0) and 91 (35.9). Anal. Calcd. for $C_{20}H_{26}O_3$: C, 76.40; H, 8.34. Found: C, 76.23; H, 8.29.

***Step 2. 17α-Methoxy-19-norpregna-4,9-diene-3,20-dione (<u>93</u>):***

A suspension of the 17$\alpha$-hydroxy dienedione (**92**, 19 g, 31.80 mmol) in $CH_3CN$ (167 mL) was stirred magnetically under nitrogen. Methyl iodide (134 mL; freshly opened) was added and a solution formed immediately. Silver oxide (8.1 g, 35.0 mmol) was added, the joints were well-greased to prevent evaporation of methyl iodide, and the flask was wrapped in foil to protect the contents from light. The mixture was brought to a gentle reflux and the reaction allowed to proceed overnight. The next morning, analysis by TLC (5% acetone in $CH_2Cl_2$) indicated virtually all the starting material had been converted to a single, less polar component. The reaction was allowed to cool to room temperature and filtered through a Celite filter cake on a sintered glass funnel. The filtrate was evaporated *in vacuo* to recover a thick syrup. Crystallization from boiling $CH_3OH$ afforded small white crystals. The crystals were collected on a Buchner funnel, triturated with cold $CH_3OH$, and dried under vacuum to recover 5.74 g. Flash chromatography of the mother liquors (5% acetone in $CH_2Cl_2$) afforded 1.69 g of additional material. The total purified product recovered was 7.43 g of **93** as white crystals in 71.1% yield; m.p. =154-155°C. FTIR (KBr, diffuse reflectance) $\nu_{max}$ 2952, 1704, 1660, 1614 and 1583 $cm^{-1}$. $^1H$ NMR (300 MHz, $CDCl_3$) $\delta$ 0.739 (s, 3 H, C18-$CH_3$), 2.164 (s, 3 H, C21-$CH_3$), 3.141 (s, 3 H, C17$\alpha$-$OCH_3$) and 5.672 (s, 1 H, C4-CH=). $^{13}C$ NMR ($CDCl_3$): $\delta$ 14.264,23.156,23.431. 23.775. 25.547. 25.753. 26.431. 27.445. 30.755, 30.793. 37.054, 39.220, 47.243, 51.348. 52.258. 96.714 (C17), 122.057 (C4), 125.228 (C10), 145.588 (C9), 157.192 (C5), 199.637 (C3) and 210.479 (C20). MS (EI) m/z (relative intensity): 328 ($M^+$, 5.8), 285 (66), 253 (64) and 213 (100). Anal. Calcd. for $C_{21}H_{28}O_3$: C, 76.79; H, 8.59. Found: C, 76.64; H, 8.59.

***Step 3. 3, 3-Ethylenedioxy-17α-methoxy-19-norpregna-5(10),9(11)-dien-20-one (94):***

Under nitrogen, a mixture of the 17α-methoxydione (**93**, 17.0 g, 51.76 mmol), triethylorthoformate (42.5 mL, 250 mmol), ethylene glycol (14 mL, 250 mmol) and *p*-toluenesulfonic acid monohydrate (0.5 g, 2.6 mmol) in dry $CH_2Cl_2$ (500 mL) was stirred at room temperature for 2 hr. After that time, TLC (2% acetone in $CH_2Cl_2$) indicated absence of starting material with formation of one major product. The reaction mixture was diluted with $CH_2Cl_2$ (~200 mL) and washed with saturated $NaHCO_3$ solution (1x), $H_2O$ (1x) and brine. The organic fractions were filtered through anhydrous sodium sulfate, combined and concentrated *in vacuo.* Recrystallization of the residue from hot methanol containing a trace of pyridine gave 16.2 g of the pure 3-ketal **94** as a white solid in 84.1% yield; m.p. = 123-125°C. FTIR (KBr, diffuse reflectance) $\nu_{max}$ 2927 and 1705 $cm^{-1}$. $^1H$ NMR (300 MHz, $CDCl_3$) δ 0.553 (s, 3 H, C18-$CH_3$), 2.147 (s, 3 H, C21-$CH_3$), 3.147 (s, 3 H, C17α-$OCH_3$), 3.983 (s, 4 H, C3-ketal) and 5.568 (br s, 1 H, C11-CH=). $^{13}C$ NMR ($CDCl_3$): δ 15.746, 23.123, 24.026, 24.570. 26.422. 27.972, 31.150, 31.298, 31.839,38.233,41.238,46.079,47.391,52.318,64.325, 64.448, 96.792, 108.131, 117.907, 126.081, 129.914 and 135.998 (signal/noise ratio obscured C20 at ~210 ppm). Anal. Calcd. for $C_{23}H_{32}O_4$: C, 74.16; H, 8.66. Found: C, 74.16; H, 8.68.

## Biological Properties of the Compounds of Formula I

### MATERIALS AND METHODS

***Statistical Analysis***

Statistical analysis was performed using standard methods and a PROPHET data management system operating on SUN Microsystems OS 4.4.1 (Bliss, Cl., The Statistics of Bioassay, New York, Academic Press (1952); Hollister, C., Nucleic Acids Research, 16:1873-1575 (1988)). Raw data, statistical and regression analysis are available.

***AntiMcGinty Test (McGinty, et aL, Endocrinology, 24:829-832 (1939))***

Immature female rabbits of the New Zealand White breed (approx. 1 kg body weight) were maintained under standard laboratory conditions and received a subcutaneous injection of 5 μg estradiol in 10% ethanol/sesame oil daily for 6 consecutive days. Twenty-four hours after the last injection of estradiol (day 7) animals underwent sterile abdominal surgery to ligate a 3-4 cm segment of both uterine horns. The experimental compound in appropriate solvent (usually 10% ethanol/sesame oil) was injected intraluminally into the ligated segment of one uterine horn and the vehicle alone into the ligated segment of the contralateral horn. Injection volume was limited to 0.1 ml,and care was taken to prevent leakage. A stimulating dose of progesterone (0.8 mg/day) was administered subcutaneously to each rabbit daily for the next three days (days 7, 8 and 9) for the purpose of inducing endometrial proliferation. All animals were sacrificed on day 10 when a segment central to the ligatures was removed and fixed in 10% neutral buffered formalin and submitted for histological processing. Five micron sections stained with hematoxylin and eosin (H&E) were evaluated microscopically for the degree of endometrial glandular proliferation according to the method of McPhail (McPhail, J. Physiol., 83: 145 (1934). The percent inhibition of endometrial proliferation for each rabbit was calculated and the mean of the group of five animals recorded.

***AntiClauberg Test* (*Clauberg, C., Zentr. Gynakol., 54:2757-2770 (1930))***

Immature female rabbits of the New Zealand White breed (approx. 1 kg body weight) were maintained under standard laboratory conditions and received a subcutaneous injection of 5 μg estradiol in 10% ethanol/sesame oil daily for 6 consecutive days. Twenty-four hours after the last dose of estradiol (day 7) animals received progesterone by subcutaneous injection (0.8 mg/day) and the experimental compound in appropriate vehicle (usually 10% ethanol/sesame oil) orally or subcutaneously for five consecutive days. One group of rabbits received progesterone only. Twenty-four hours after the last dose all animals were sacrificed for removal of the uterus which was cleaned of all fat and connective tissue, weighed to the nearest 0.2 mg and placed in 10% neutral buffered formalin for subsequent histological processing. Five micron sections stained with hematoxylin and eosin (H&E) were evaluated microscopically for the degree of endometrial glandular proliferation according to the method of McPhail (McPhail, *supra).* The percent inhibition of endometrial proliferation at each dose level of the experimental compound was derived by comparison with the progesterone-stimulated animals alone.

***Postcoital Test***

Adult female rats of the Sprague-Dawley strain were maintained under standard laboratory conditions, 14 hours

of light and 10 hours of darkness each day and cohabited with proven fertile males when in proestrus. Sperm-positive animals were randomly assigned to control and experimental groups. The day vaginal sperm were found in vaginal washings constituted day 0 of gestation. Rats received experimental compounds or vehicle (control) daily by the oral route on days 0-3 or 4-6 and were sacrificed between days 10 and 17 to record the number and condition of conceptuses.

***Antiovulatory Test***

Immature female rats of the Sprague-Dawley strain weighing 200 to 250 g were maintained under standard laboratory conditions, 14 hours of light and 10 hours of darkness each day. Vaginal washings were obtained daily and evaluated microscopically to establish the estrous cycle of each animal. Animals exhibiting two consecutive four-day cycles were used in the test. Each dose group consisted of eight rats and one group served as the vehicle control. Animals were dosed at noon on the day of proestrus and sacrificed 24 hours later when ova can usually be visualized in the distended ampulla of the oviduct using a dissecting microscope. The oviducts were excised, an incision made in the distended ampulla and the ova teased out in a drop of water on a microscope slide so that the number shed could be counted. Historically, control animals shed between 12 and 14 ova during each estrous cycle. Agents which inhibit ovulation usually exhibit an "all or none" effect; it is rare that ovulation is "partially" inhibited. Treatment groups were compared with the control group using a 95% contingency table or the $ED_{100}$ was established with additional dose levels.

***Relative Binding Affinities for the Progesterone and Glucocorticoid Receptors***

Uteri and thymus glands were obtained from estradiol-primed immature female rabbits of the New Zealand White strain for preparation of cytosols for the progesterone and glucocorticoid receptor assays, respectively. Tissues were excised and immediately placed in ice cold TEGDM buffer (10 mM Tris, pH 7.4; 1.5 mM EDTA; 10% glycerol vol/vol/; 1 mM dithiothreitol [DTT]; and 20 mM sodium molybdate). The tissues were dissected free of connective tissue and fat, weighed and minced finely. Minced tissues were homogenized in 3 volumes TEGDM/gm with four 10 second bursts of a VirTis Cyclone set at half maximum speed with a 30 second cooling period (in ice) between bursts. Homogenates were centrifuged at 109,663 g at 4°C for 1 hour to yield the soluble cytosol fraction. Aliquots of cytosol were snap frozen and stored at -75°C.

All binding assays were carried out at 2-6°C for 16-18 hours. The following radioactive ligands were used: [1,2-$^3$H(N)]-progesterone (50.0 Ci/mmole) for the progesterone receptor (PR). (6,7-$^3$H(N)-dexamethasone (39.2 Ci/mmole) for the glucocorticoid receptor (GR) and [2,4,6,7-$^3$H(N)]-estradiol for the estrogen receptor. For the progesterone receptor RBA assays 0.02 ml uterine cytosol or TEDGM buffer, 0.05 ml of various concentrations of test compounds or progesterone, 0.13 ml TEGDM buffer and 0.05 ml [$^3$H]-progesterone were added to duplicate tubes. For the glucocorticoid receptor RBA assays 0.1 ml thymus cytosol or TEDGM buffer, 0.05 ml of various concentrations of test compounds or dexamethasone, 0.05 ml TEGDM buffer and 0.05 ml [$^3$H]-dexamethasone were added to duplicate tubes. For the estrogen receptor RBA assays 0.05 ml uterine cytosol, 0.1 ml TEGDM buffer, 0.05 ml of various concentrations of test compounds or estradiol and 0.05 ml [$^3$H]-estradiol were added to duplicate tubes. The concentrations of the test compounds, progesterone, dexamethasone and estradiol ranged from 0.05 to 100 nM and the concentrations of the competitors ranged from 0.5 to 500 nM. Total binding was measured at radioligand concentrations of 3.5 nM and nonspecific binding was measured in the presence of a 200-fold excess of unlabeled progesterone (PR), dexamethasone (GR) or diethylstilbestrol (ER), respectively.

In all incubations bound and free ligand were separated using dextra-coated charcoal (DCC). A 0.1 ml aliquot of DCC (0.5% charcoal/0.05% Dextran T-70) was added to each tube. The tubes were vortexed and incubated on ice for 10 minutes. Five-tenths ml TEG buffer (without DTT or molybdate) was then added to all tubes to improve supernatant recovery following centrifugation. The charcoal was pelleted by centrifugation at 2,100 g for 15 minutes at 4°C. The supernatants containing the [$^3$H]-steroid receptor complexes were decanted into vials containing 4 ml Optifluor (Packard Instrument Co.), vortexed, equilibrated in a liquid scintillation counter for 30 minutes and then counted for 2 minutes. This provided the quantity of receptor bound [$^3$H]-steroid at each competitor concentration.

The standard curves and the $EC_{50}$ (Effective Concentration) for each standard curve and curve for each test compound was determined by entering the counting data (receptor bound [$^3$H]-progesterone, [$^3$H]-dexamethasone or [$^3$H]-estradiol) into a four parameter sigmoidal computer program (RiaSmart® Immunoassay Data Reduction Program, Packard Instrument Co., Meriden, Connecticut. The RBA for each test compound was calculated using the following equation:

$$RBA = \frac{EC_{50}\ Standard}{EC_{50}\ Test\ Compound} X\ 100$$

where $EC_{50}$ Standard = molar concentration of unlabeled progesterone, dexamethasone or estradiol required to decrease bound [$^3$H]-progesterone (PR), [$^3$H]-dexamethasone (GR) or [$^3$H]-estradiol to 50% of the respective buffer control (100% bound radioligand) and $EC_{50}$ Test Compound = molar concentration of test compound required to decrease bound [$^3$H]-progesterone (PR), [$^3$H]-dexamethasone (GR) or [$^3$H]-estradiol to 50% of the respective buffer control (100% bound radioligand).

**RESULTS**

**EXAMPLE 1**

Results of the antiMcGinty and oral antiClauberg tests as well as the relative binding affinities are shown in Tables 1 and 2. Compared to the lead compound (CDB-2914,21-H), the 21-acetoxy (**15**) and the 21-methoxy (**38**) analogs exhibited 2.79 and 3.61 times, respectively, the antiprogestational potency as assessed by the oral antiClauberg test with a substantial reduction in glucocorticoid binding affinity. Further, the results of the antiMcGinty test of the 21-acetoxy analog (15) following intraluminal administration closely paralleled those observed in the antiClauberg test following oral dosing. Since mifepristone (CDB-2477) is frequently used as a reference standard, Table 3 contains data comparing the antiprogestational activity and relative binding affinity for the progesterone and glucocorticoid receptors of CDB-2914 with this standard. Recent studies have shown a good correlation between relative binding affinity for the glucocorticoid receptor and a biological test based upon the antagonism of dexamethasone-induced thymus involution in adrenalectomized male rats.

The halogenated analogs (**13, 14A, 14B**) did not show significant differences in antiprogestational activity nor relative binding affinity to the progesterone receptor from the lead compound, CDB-2914. Other 21-substituted analogs generally exhibited reduced antiprogestational activity with the exception of the cypionate (**40**) which was about 50% more potent in the antiClauberg test. This may be due to hydrolysis to the corresponding 21-hydroxy compound. However, the presence of additional bulkiness at position 21 does not always favor an increase in biological activity (see **14B**) and enhanced relative binding affinity for the progesterone receptor was not necessarily indicative of greater antiprogestational activity (see **12**). Thus the window of opportunity for enhanced antiprogestational activity with a reduction in relative binding affinity for the glucocorticoid receptor for 21-substituted analogs of the lead compound (CDB-2914) is highly restricted and was identified only after numerous analogs had been synthesized and tested.

**Table 1**

| ANTIPROGESTATIONAL ACTIVITY AND RELATIVE BINDING AFFINITY FOR THE PROGESTERONE AND GLUCOCORTICOID RECEPTORS | | | | | |
|---|---|---|---|---|---|
| COMPOUND | | ANTIPROGESTATIONAL[1] | | RELATIVE BINDING AFFINITY[2] | |
| Appln. No. | CDB No. | AntiMcGinty | AntiClauberg | Progesterone | Glucocorticoid |
| 69B | 2914 | 100 | 100 | 122 | 114 |
| 12 | 4062 | 26 | 29 | 261 | 32 |
| 13 | 4058 | 103 | 80 | 125 | 109 |
| 14A | 3876 | 75 | 68 | 127 | 90 |
| 14B | 4031 | 71 | | 130 | 175 |
| 15 | 4059 | 300 | 279 | 103 | 51 |
| 16 | 4102 | >2 | | 6 | 77 |
| 17 | 4101 | 65 | | 37 | 54 |
| 28 | 4030 | 32 | | 129 | 126 |
| 38 | 4124 | | 361 | 103 | 52 |

(continued)

| ANTIPROGESTATIONAL ACTIVITY AND RELATIVE BINDING AFFINITY FOR THE PROGESTERONE AND GLUCOCORTICOID RECEPTORS | | | | | |
|---|---|---|---|---|---|
| COMPOUND | | ANTIPROGESTATIONAL[1] | | RELATIVE BINDING AFFINITY[2] | |
| Appln. No. | CDB No. | AntiMcGinty | AntiClauberg | Progesterone | Glucocorticoid |
| 40 | 4125 | | 155 | 74 | 37 |
| 41 | 4152 | | 140 | 62 | 71 |
| 46 | 4167 | | 130-210 | 83 | 46 |

[1]Antiprogestational Activity AntiMcGinty: see text; CDB-2914 = 100 (assigned) AntiClauberg, oral:see text; CDB-2914 = 100 (assigned)
[2]Relative Binding Affinity Progesterone receptor (estrogen-primed rabbit uterus) progesterone = 100% Glucocorticoid receptor (estrogen-primed rabbit thymus) dexamethasone = 100%

**Table 2**

| CDB NO[5]. | COMPOUND NO. | BINDING AFFINITY[1] | | BIOLOGICAL ACTIVITY | | |
|---|---|---|---|---|---|---|
| | | Progester | Glucocortic | antiClauberg[2] | Postcoitat[3] | Antiowlator[4] |
| 2914 | 69B | 122(234) | 114 | 100 | 2 | 1 |
| 3875 | 69A | 164 | 30 | 97 | | |
| 3247 | 69C | 91 | 49 | ~10 | 2* | |
| 3248 | 69D | 40 | 89 | weak (subcu) | inactive @2* | |
| 4243 | 91 | 171 | 59 | inactive | | |
| 4418 | 70 | 79 | /2 | ~25 | | |
| 4363 | 71 | 123 (203) | 20 | 253 | 0.5 | >16 |
| 4399 | 72 | 109 | 110 | 35 | | |
| 4176 | 74 | 131 | 32 | <10 | | |
| 4324 | 97a | 120 | 52 | 110 | | |
| 4398 | 97b | 47 | 38 | 99 | | |
| 4455 | 106a | | | | | |
| 4241 | 106b | 136(172) | 14 | 34 | | |
| 4400 | 113A | 117 (237) | 62 | 229 | | |
| 4454 | 113B | 59 | 34 | | | |
| 4417 | 113c | 63 | 45 | 70 | | |
| 4239 | 123a | 174(140) | 11 | 45-83 | | |
| 4416 6 | 123b | 64 | 45 | 77 | | |
| 4393 | 139 | 30 | 79 | inactive | | |
| 4247 | 126a | 95 | 43 | 170 | | |
| 4362 | 126b | 76 | 15 | 125 | | |
| 4374 | 126c | 68 | 67 | 224 | | |
| 4361 | 129 | 155 | 20 | 303 | | |
| 4306 | 133 | 82 | 13 | 95 | | |

(continued)

| CDB NO[5]. | COMPOUND NO. | BINDING AFFINITY[1] | | BIOLOGICAL ACTIVITY | | |
|---|---|---|---|---|---|---|
| | | Progester | Glucocortic | antiClauberg[2] | Postcoitat[3] | Antiowlator[4] |
| 4352 | 138 | 63 | 14 | 57 | | |

[1]Progesterone receptor (estrogen-primed rabbit uterus); progesterone = 100% Figure in () is relative binding affinity of the human isoform A progesterone receptor Glucocorticoid receptor (estrogen-primed rabbit thymus) dexamethasone = 100%.
[2]antiClauberg - oral except where indicated; CDB-2914 = 100 (assigned).
[3]Postcoital - oral, rat $MED_{100}$ (mg/day) days 0-3 or *days 4-6 subcu; day sperm in vaginal washings = day 0.
[4]Antiovulatory - oral, rat $MED_{100}$(mg) single dose at noon on day of proestrus.
[5] The claimed compounds are compounds 113a, 126b and 129.

**Table 3**

| RELATIVE BINDING AFFINITIES AND ANTIPROGESTATION ACTIVITY OF CDB-2914 AND MIFEPRISTONE (CDB-2477) | | | | |
|---|---|---|---|---|
| DRUG | RELATIVE BINDING AFFINITY | | ANTIPROGESTATIONAL ACTMTY | |
| | PROGESTERONE[1] | GLUCOCORTICOID[2] | ANTIMCGINITY[3] | ANTICLAUBERG[4] |
| CDB-2914 | 114-(n=18) | 127-24 (n=12) | 0.56 | 3.27 |
| CDB-2477 | 150-17 (n=11) | 221-35 (n=6) | 1.0 (assigned) | 1.0 (assigned) |

[1]Progesterone = 100%; immature estrogen-primed rabbit uterus
[2]Dexamethasone = 100%; immature estrogen-primed rabbit thymus
[3]Intraluminal administration to estrogen-primed immature rabbits; CDB-2477 = 1.0 (assigned)
[4]Oral administration to estrogen-primed immature rabbits; CDB-2477 = 1.0 (assigned)

**EXAMPLE 2**

***AntiClauberg***

Data from antiClauberg tests following oral administration are shown in Tables 1 and 2. Compounds **15, 38, 40, 41, 46, 71, 97a, 113a, 126a, 126b, 126c** and **129** exhibited greater activity than the standard, **69B**. Previous studies have shown that 69B is significantly more potent than mifepristone (3.27 X; 95% C.I. = 1.41-7.58) in this test. Compounds **15, 38, 71** and **129** represent four of the most potent antiprogestational compounds known, and their low binding affinity for the glucocorticoid receptor would predict minimal antiglucocorticoid activity.

***Postcoital***

Compound **71** exhibited about four times the postcoital contraceptive activity of the standard, compound **69B**, following oral administration on days 0-3 of gestation.

***Antiovulatory***

Compound **71** was not fully active at a dose level 16 times the $MED_{100}$ for the standard, compound **69B,** and compound **113a** exhibited only about 6% of the antiovulatory activity of the standard.

***Relative Binding Affinity for the Progesterone and Glucocorticoid Receptors***

Relative binding affinities for the progesterone receptor (estrogen-primed rabbit uterine cytosol) and glucocorticoid receptor (estrogen-primed rabbit thymic cytosol) are shown in Table 1. Several compounds were also tested for binding affinity for the human isoform A progesterone receptor. Compounds **12, 13, 14A, 14B, 15, 28, 38, 69A, 91, 71, 72, 73, 97a, 106b, 113a, 113d, 122b** and **129** showed binding affinities greater than that observed for the standard, compound **69B**. On the other hand, most of the compounds tested exhibited reduced binding affinity for both the progesterone and the glucocorticoid receptor.

**DISCUSSION**

Many members of a series of derivatives of 19-norprogesterone possess potent antiprogestational activity following oral administration in experimental animals. They exhibit high binding affinity for the progesterone receptor (rabbit uterine) and only modest relative binding affinity for the glucocorticoid receptor (rabbit thymus). This is reflected in standard antiprogestational assays showing strong inhibition of progesterone-induced alterations of rabbit uterine endometrium. It is anticipated that the reduced binding affinity for the glucocorticoid receptor will reflect diminished biological antiglucocorticoid activity.

Table 3 compares the relative binding affinity for the progesterone and glucocorticoid receptors as well as the antiprogestational activity as measured by antiClauberg and antiMcGinty tests for the standard, compound **69B**, and mifepristone (CDB-2477). Mifepristone exhibited greater binding affinity for both receptor proteins and was more potent than the standard, compound **69B**, in the antiMcGinty test. However, the standard was 3 times as potent as mifepristone in the antiClauberg test following oral administration. This finding has not been satisfactorily explained, but may be due to the differential pharmacokinetics of these two steroids following oral administration. Higher blood levels of **69B** have been observed following oral administration to several species, thus indicating a greater oral bioavailable for the standard.

Antiprogestational agents including mifepristone are known to prevent implantation in the rat (Dao, B., et al., Contraception, 54:243-258 (1996); Reel, J., et al., Contraception, 58:129-136 (1998)), guinea pig (Badsta, M., et al., Am. J Obstet. Gynecol., 165:82-86 (1991), and man (Baulieu, E., Clinical Applications of Mifepristone (RU486) and Other Antiprogestins (Donaldson, M., Dorflinger, L., Brown, S. and Benet, L. (eds.), National Academy Press, pp. 72-119 (1993)). Compound **71** was four times as potent as the standard, compound **69B**, in preventing pregnancy when orally administered on days 0-3 of presumptive gestation. Curiously, compound **71** was only about 5% as potent as the standard in inhibiting ovulation. Both compound **69B** and mifepristone have been shown to inhibit ovulation in the rat (Dao, *et al., supra*), and mifepristone has been shown to affect ovulation in human subjects (Baulieu, *et al., supra*). Compound **69B** has been shown to affect both follicular development and ovulation as well as endometrial maturation in human subjects following a single oral dose (unpublished data).

Compound **113a** exhibited high binding affinity for both the rabbit progesterone receptor (isoform B) and the human progesterone receptor (isoform A). This was reflected in potent antiprogestational activity *in vivo* where it was more than twice as active at the standard, compound **69B**. It also showed reduced binding affinity for the glucocorticoid receptor and was about half as effective as compound 69B in preventing pregnancy in the postcoital test. Strangly, this compound was only 6% as active as the standard in inhibiting ovulation. Thus, compound **113a** may represent an antiprogestational steroid with high tissue specificity.

**Claims**

1. A compound having the general formula:

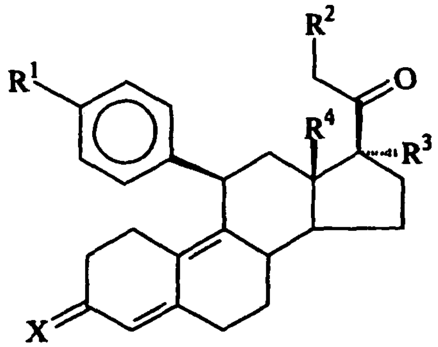

wherein:

$R^1$ is $-N(CH_3)_2$;
$R^4$ is methyl;
X is = O; and

$R^2$ and $R^3$ are both $-OCH_3$, or $R^3$ is acetoxy and $R^2$ is a member selected from the group consisting of $-OCH=CH_2$ and $-OC(O)R^6$ wherein $R^6$ is $-CH_2OCH_3$.

**2.** The compound in accordance with claim 1, wherein:

$R^1$ is $-N(CH_3)_2$;
$R^2$ is $-OCH_3$ ;
$R^3$ is $-OCH_3$;
$R^4$ is methyl; and
X is =O.

**3.** The compound in accordance with claim 1, wherein:

$R_1$ is $-N(CH_3)_2$;
$R_2$ is $-OC(O)CH_2OCH_3$;
$R_3$ is acetoxy;
$R_4$ is methyl; and
X is =0.

**4.** The compound in accordance with claim 1, wherein:

$R_1$ is $-N(CH_3)_2$;
$R_2$ is $-OCH=CH_2$;
$R_3$ is acetoxy;
$R_4$ is methyl; and
X is =O.

**5.** A pharmaceutical composition comprising an effective amount of a compound in accordance with any one of claims 1 to 4 and a pharmaceutically acceptable excipient.

**6.** A compound in accordance with any one of claims 1 to 4, for use in producing an antiprogestational effect in a patient.

**7.** A compound in accordance with any one of claims 1 to 4, for use in:

(a) inducing menses;
(b) treating endometriosis;
(c) treating dysmenorrhea;
(d) treating an endocrine hormone-dependent tumor,
(e) treating meningiomas;
(f) treating uterine fibroids;
(g) inhibiting uterine endometrial proliferation; or
(h) inducing labor.

**8.** Use of a compound in accordance with any one of claims 1 to 4 for the manufacture of a medicament for.

(a) inducing menses;
(b) treating endometriosis;
(c) treating dysmenorrhea;
(d) treating an endocrine hormone-dependent tumor;
(e) treating meningiomas;
(f) treating uterine fibroids;
(g) inhibiting uterine endometrial proliferation; or
(h) inducing labor.

**Patentansprüche**

**1.** Verbindung mit der allgemeinen Formel:

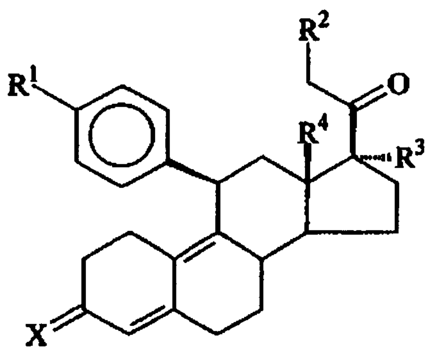

worin

$R^1$ $-N(CH_3)_2$ ist;
$R^4$ Methyl ist;
X =O ist; und
$R^2$ und $R^3$ beide $-OCH_3$ sind, oder $R^3$ Acetoxy ist und $R^2$ ein Vertreter ist, der aus der Gruppe gewählt wird, die aus $-OCH=CH_2$ und $-OC(O)R^6$ besteht, worin $R^6$ $-CH_2OCH_3$ ist.

**2.** Verbindung gemäß Anspruch 1, worin:

$R^1$ $-N(CH_3)_2$ ist;
$R^2$ $-OCH_3$ ist;
$R^3$ $-OCH_3$ ist;
$R^4$ Methyl ist; und
X =O ist.

**3.** Verbindung gemäß Anspruch 1, worin:

$R^1$ $-N(CH_3)_2$ ist;
$R^2$ $-OC(O)CH_2OCH_3$ ist;
$R^3$ Acetoxy ist;
$R^4$ Methyl ist; und
X =O ist.

**4.** Verbindung gemäß Anspruch 1, worin:

$R^1$ $-N(CH_3)_2$ ist;
$R^2$ $-OCH=CH_2$ ist;
$R^3$ Acetoxy ist;
$R^4$ Methyl ist; und
X =O ist.

**5.** Pharmazeutische Zusammensetzung, umfassend eine wirksame Menge einer Verbindung gemäß mindestens einem der Ansprüche 1 bis 4 und einen pharmazeutisch annehmbaren Arzneimittelträger.

**6.** Verbindung gemäß mindestens einem der Ansprüche 1 bis 4 zur Verwendung bei der Hervorrufung eines antiprogestationalen Effektes in einem Patienten.

**7.** Verbindung gemäß mindestens einem der Ansprüche 1 bis 4 zur Verwendung für das:

(a) Induzieren von Menstruationen;
(b) Behandeln von Endometriose;
(c) Behandeln von Dysmenorrhoe;
(d) Behandeln eines endokrinen Horman-abhängigen Tumors;
(e) Behandeln von Meningiomata;

(f) Behandeln von Uterusfibroiden;
(g) Inhibieren der Uterus-endometrialen Proliferation; oder
(h) Induzieren von Wehen.

**8.** Verwendung einer Verbindung gemäß mindestens einem der Ansprüche 1 bis 4 zur Herstellung eines Medikamentes für das:

(a) Induzieren von Menstruationen;
(b) Behandeln von Endometriose;
(c) Behandeln von Dysmenorrhoe;
(d) Behandeln eines endokrinen Horman-abhängigen Tumors;
(e) Behandeln von Meningiomata;
(f) Behandeln von Uterusfibroiden;
(g) Inhibieren der Uterus-endometrialen Proliferation; oder
(h) Induzieren von Wehen.

## Revendications

**1.** Composé de formule générale :

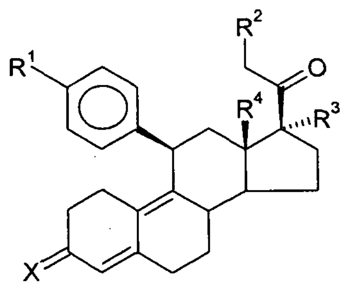

dans laquelle

- $R^1$ représente un groupe de formule $-N(CH_3)_2$,
- $R^4$ représente un groupe méthyle,
- X représente un atome d'oxygène,
- et $R^2$ et $R^3$ représente chacun un groupe méthoxy, ou bien $R^3$ représente un groupe acétyloxy et $R^2$ représente un groupe de formule $-OCH=CH_2$ ou $-OC(O)R^6$ où $R^6$ représente un groupe de formule $-CH_2OCH_3$.

**2.** Composé conforme à la revendication 1, dans lequel :

- $R^1$ représente un groupe de formule $-N(CH_3)_2$,
- $R^2$ représente un groupe méthoxy,
- $R^3$ représente un groupe méthoxy,
- $R^4$ représente un groupe méthyle,
- et X représente un atome d'oxygène.

**3.** Composé conforme à la revendication 1, dans lequel :

- $R^1$ représente un groupe de formule $-N(CH_3)_2$,
- $R^2$ représente un groupe de formule $-OC(O)CH_2OCH_3$,
- $R^3$ représente un groupe acétyloxy,
- $R^4$ représente un groupe méthyle,
- et X représente un atome d'oxygène.

**4.** Composé conforme à la revendication 1, dans lequel :

- $R^1$ représente un groupe de formule $-N(CH_3)_2$,
- $R^2$ représente un groupe de formule $-OCH=CH_2$,
- $R^3$ représente un groupe acétyloxy,
- $R^4$ représente un groupe méthyle,
- et X représente un atome d'oxygène.

**5.** Composition pharmaceutique comprenant, en une quantité efficace, un composé conforme à l'une des revendications 1 à 4, et un excipient pharmacologiquement acceptable.

**6.** Composé conforme à l'une des revendications 1 à 4, conçu pour servir à produire un effet antiprogestatif chez un patient.

**7.** Composé conforme à l'une des revendications 1 à 4, conçu pour servir :

a) à provoquer les règles,
b) à traiter une endométriose,
c) à traiter une dysménorrhée,
d) à traiter une tumeur dépendante d'une hormone endocrine,
e) à traiter un méningiome,
f) à traiter un fibrome utérin,
g) à inhiber une prolifération endométriale,
h) ou à provoquer le travail d'accouchement.

**8.** Emploi d'un composé conforme à l'une des revendications 1 à 4 en vue de la fabrication d'un médicament conçu :

a) pour provoquer les règles,
b) pour traiter une endométriose,
c) pour traiter une dysménorrhée,
d) pour traiter une tumeur dépendante d'une hormone endocrine,
e) pour traiter un méningiome,
f) pour traiter un fibrome utérin,
g) pour inhiber une prolifération endométriale,
h) ou pour provoquer le travail d'accouchement.

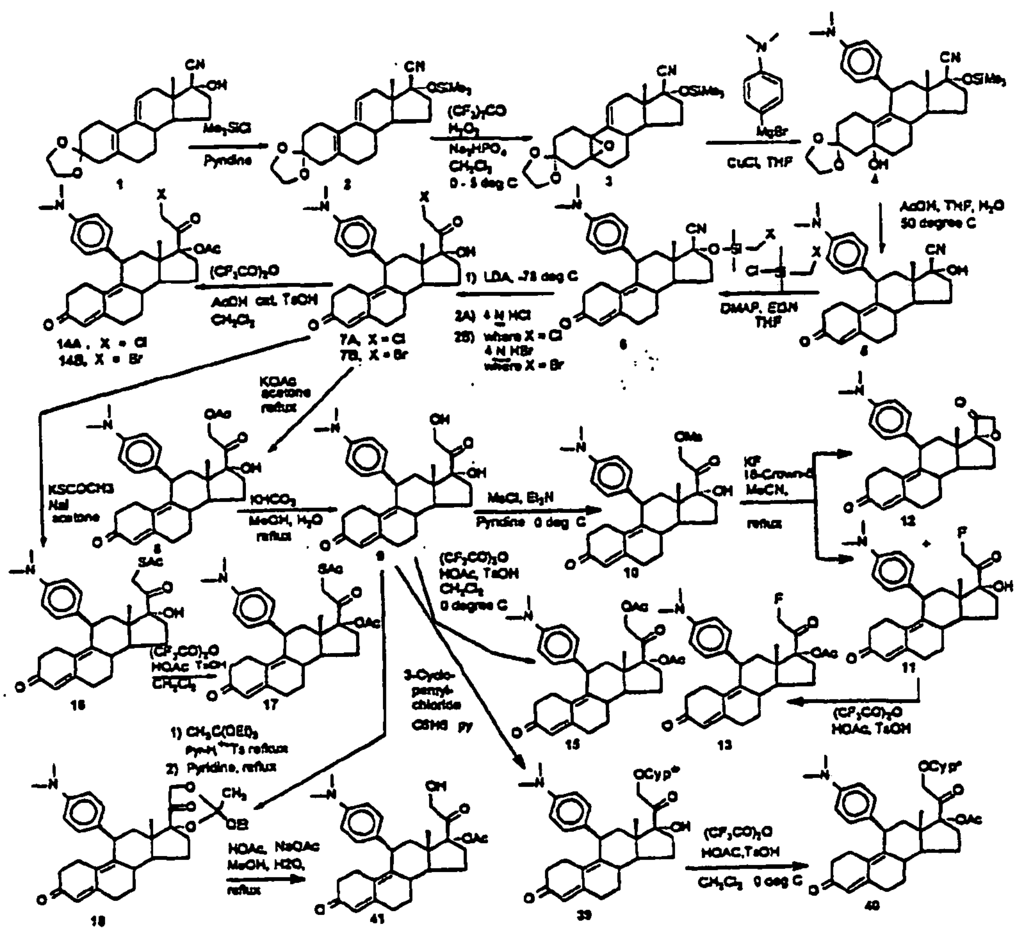

Me3SiCl
Pyridine
(CF3)2CO
H2O2
Na2HPO4
CH2Cl2
0 - 5 deg C
MgBr
CuCl, THF
AcOH, THF, H2O
50 degree C
DMAP, Et3N
THF
1) LDA, -78 deg C
2A) 4 N HCl
2B) where X = Cl
4 N HBr
where X = Br
(CF3CO)2O
AcOH cat. TsOH
CH2Cl2
14A, X = Cl
14B, X = Br
7A, X = Cl
7B, X = Br
KOAc
acetone
reflux
KSCOCH3
NaI
acetone
KHCO3
MeOH, H2O
reflux
MsCl, Et3N
Pyridine 0 deg C
KF
18-Crown-6
MeCN,
reflux
(CF3CO)2O
HOAc, TsOH
CH2Cl2
0 degree C
(CF3CO)2O
HOAc TsOH
CH2Cl2
3-Cyclo-pentyl-chloride
C6H6 py
(CF3CO)2O
HOAc, TsOH
1) CH3C(OEt)3
Py-H+ -Ts reflux
2) Pyridine, reflux
HOAc, NaOAc
MeOH, H2O,
reflux
(CF3CO)2O
HOAC,TsOH
CH2Cl2 0 deg C
1
2
3
4
5
6
8
9
10
11
12
13
15
16
17
18
39
40
41


FIGURE 1

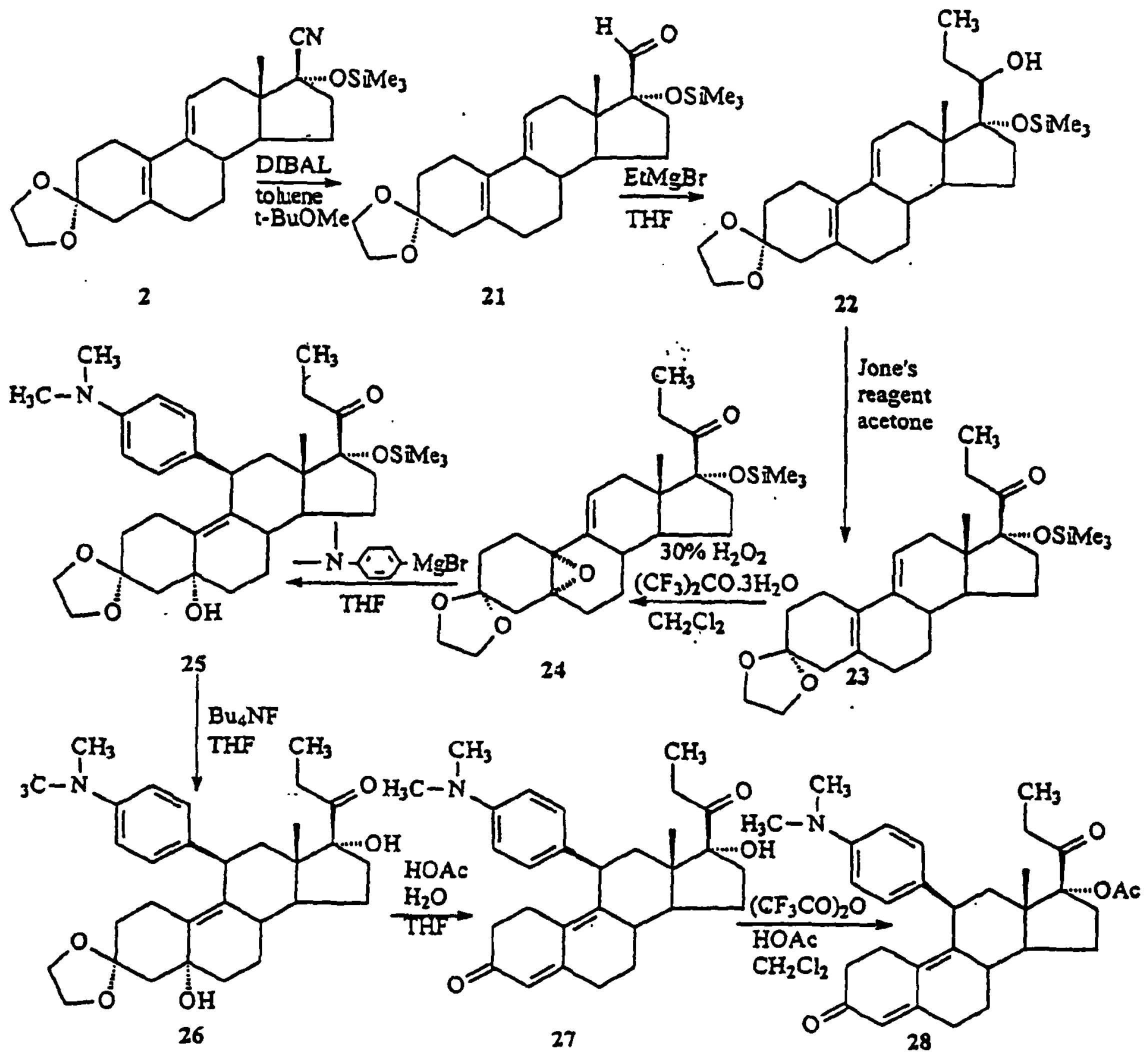
DIBAL
toluene
t-BuOMe
EtMgBr
THF
Jone's
reagent
acetone
30% $H_2O_2$
$(CF_3)_2CO.3H_2O$
$CH_2Cl_2$
THF
$Bu_4NF$
THF
HOAc
$H_2O$
THF
$(CF_3CO)_2O$
HOAc
$CH_2Cl_2$
2
21
22
23
24
25
26
27
28

FIGURE 2

1

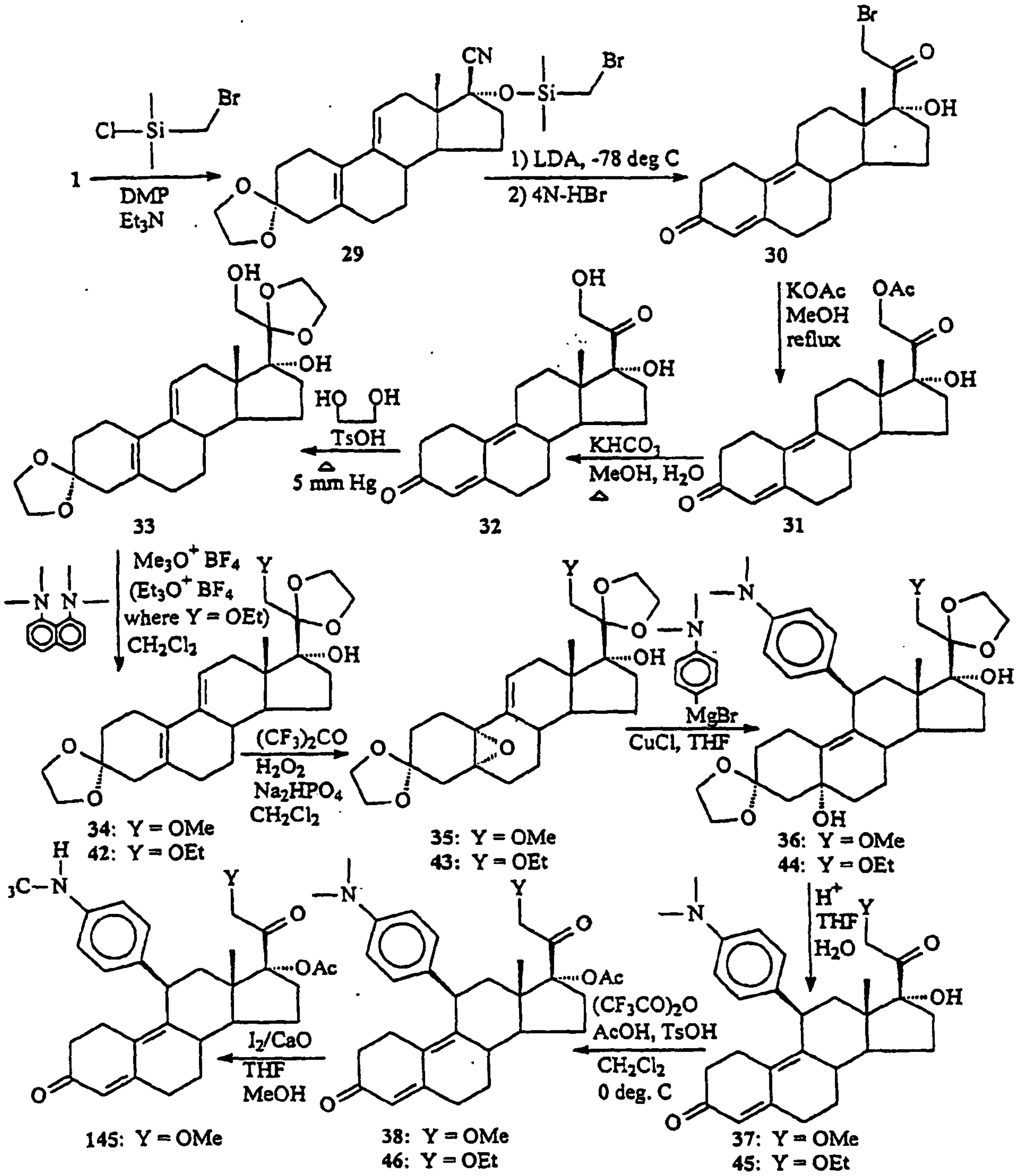


29

30

31

32

33

34: Y = OMe
42: Y = OEt

35: Y = OMe
43: Y = OEt

36: Y = OMe
44: Y = OEt

37: Y = OMe
45: Y = OEt

38: Y = OMe
46: Y = OEt

145: Y = OMe

FIGURE 3

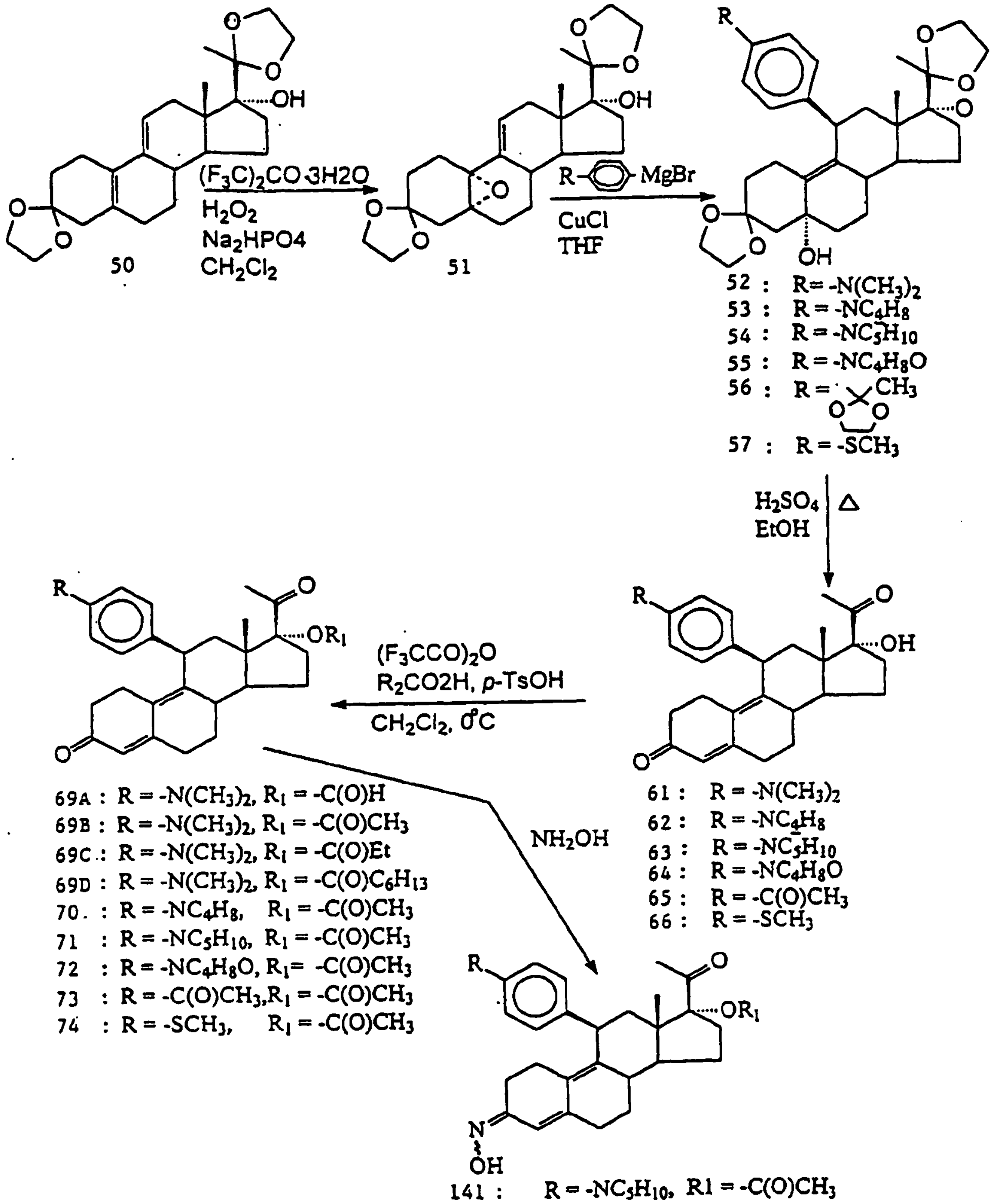


50

51

52 : $R = -N(CH_3)_2$
53 : $R = -NC_4H_8$
54 : $R = -NC_5H_{10}$
55 : $R = -NC_4H_8O$
56 : R =
57 : $R = -SCH_3$

61 : $R = -N(CH_3)_2$
62 : $R = -NC_4H_8$
63 : $R = -NC_5H_{10}$
64 : $R = -NC_4H_8O$
65 : $R = -C(O)CH_3$
66 : $R = -SCH_3$

69A : $R = -N(CH_3)_2$, $R_1 = -C(O)H$
69B : $R = -N(CH_3)_2$, $R_1 = -C(O)CH_3$
69C : $R = -N(CH_3)_2$, $R_1 = -C(O)Et$
69D : $R = -N(CH_3)_2$, $R_1 = -C(O)C_6H_{13}$
70 : $R = -NC_4H_8$, $R_1 = -C(O)CH_3$
71 : $R = -NC_5H_{10}$, $R_1 = -C(O)CH_3$
72 : $R = -NC_4H_8O$, $R_1 = -C(O)CH_3$
73 : $R = -C(O)CH_3$, $R_1 = -C(O)CH_3$
74 : $R = -SCH_3$, $R_1 = -C(O)CH_3$

141 : $R = -NC_5H_{10}$, $R1 = -C(O)CH_3$

Figure 4

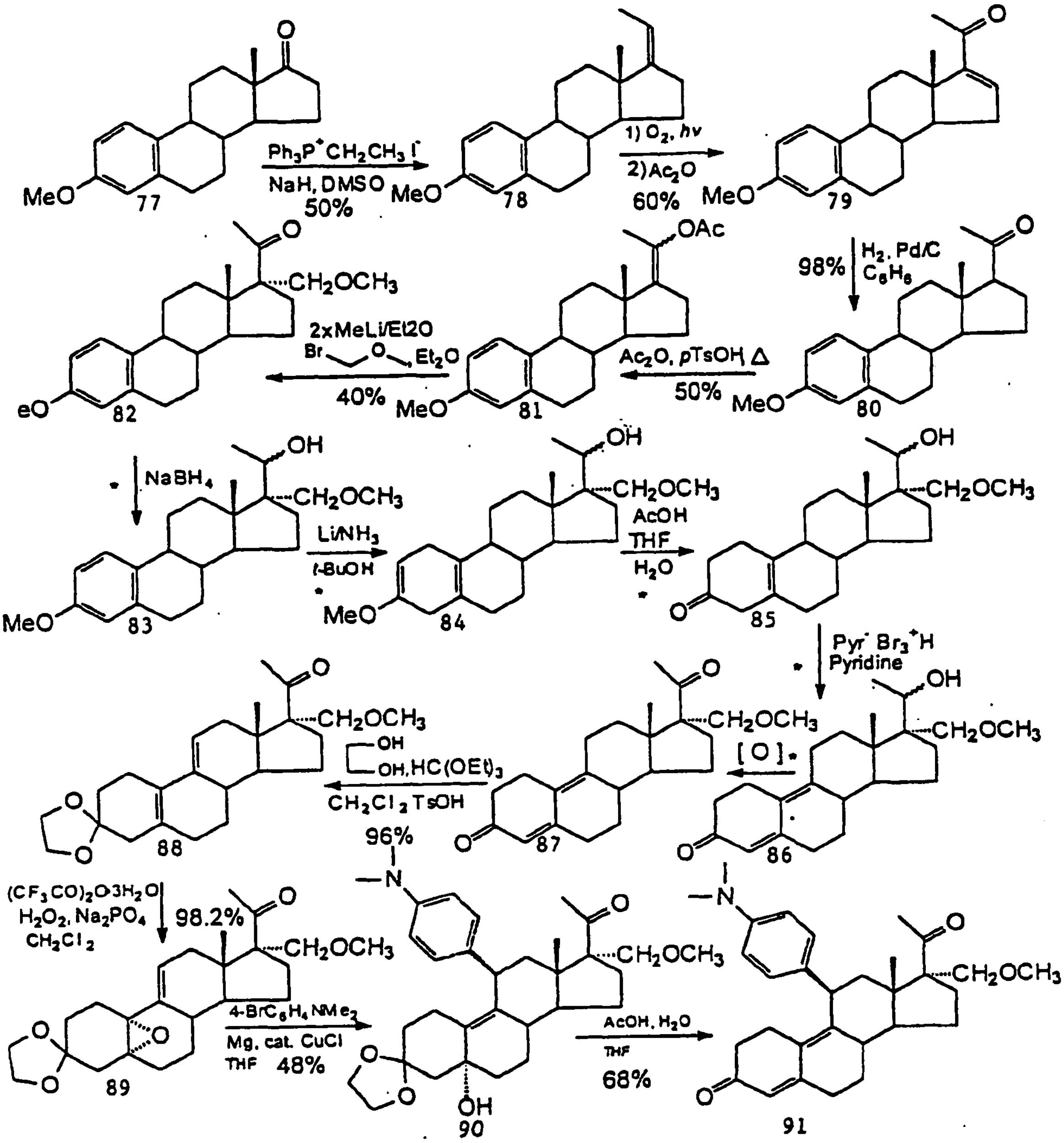


* Yield from 83 to 86 is 37%

Figure 5

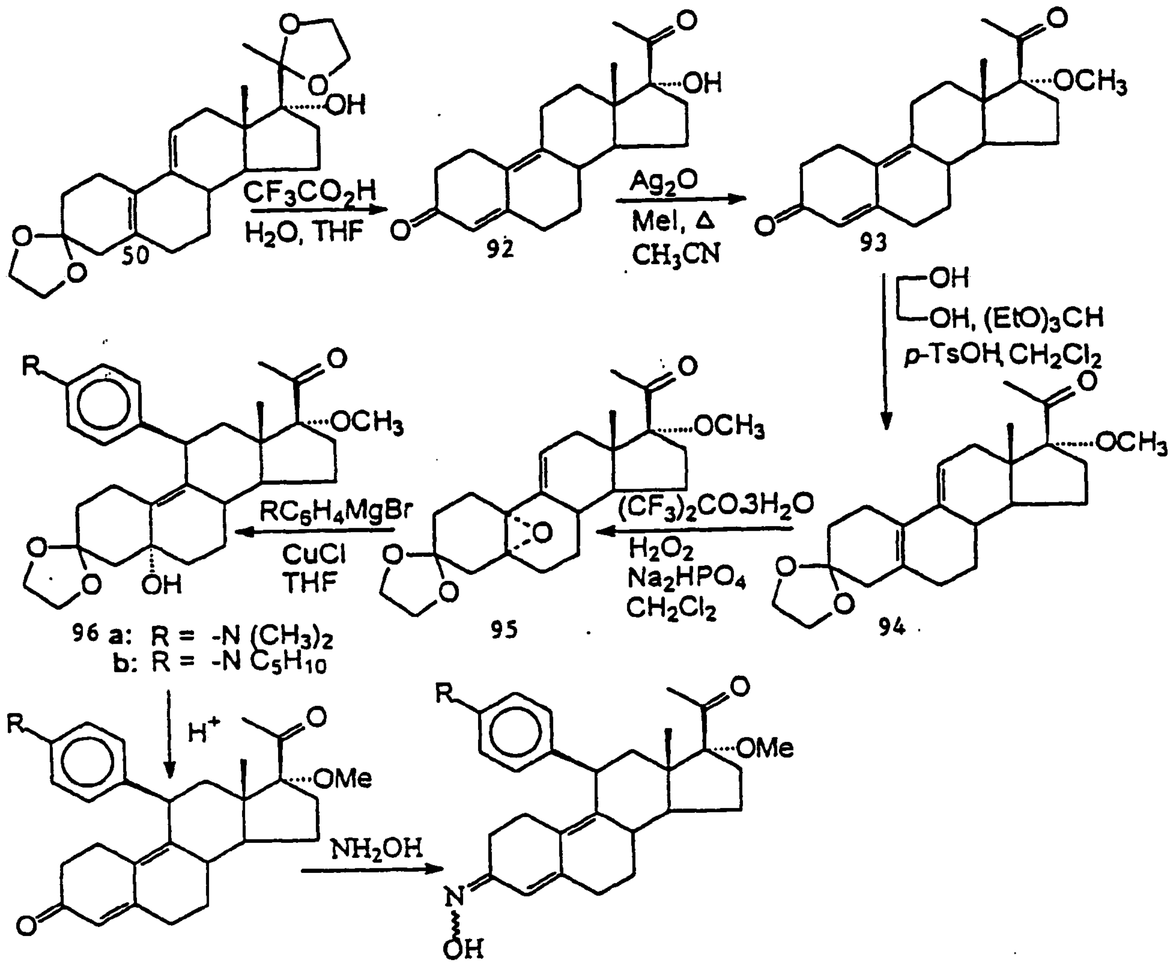


50, 92, 93, 94, 95

96 a: R = -N (CH3)2
b: R = -N C5H10

97 a: R = -N (CH3)2
b: R = -N C5H10

142 a: R = -N(CH3)2
b: R = -N C5H10

Figure 6

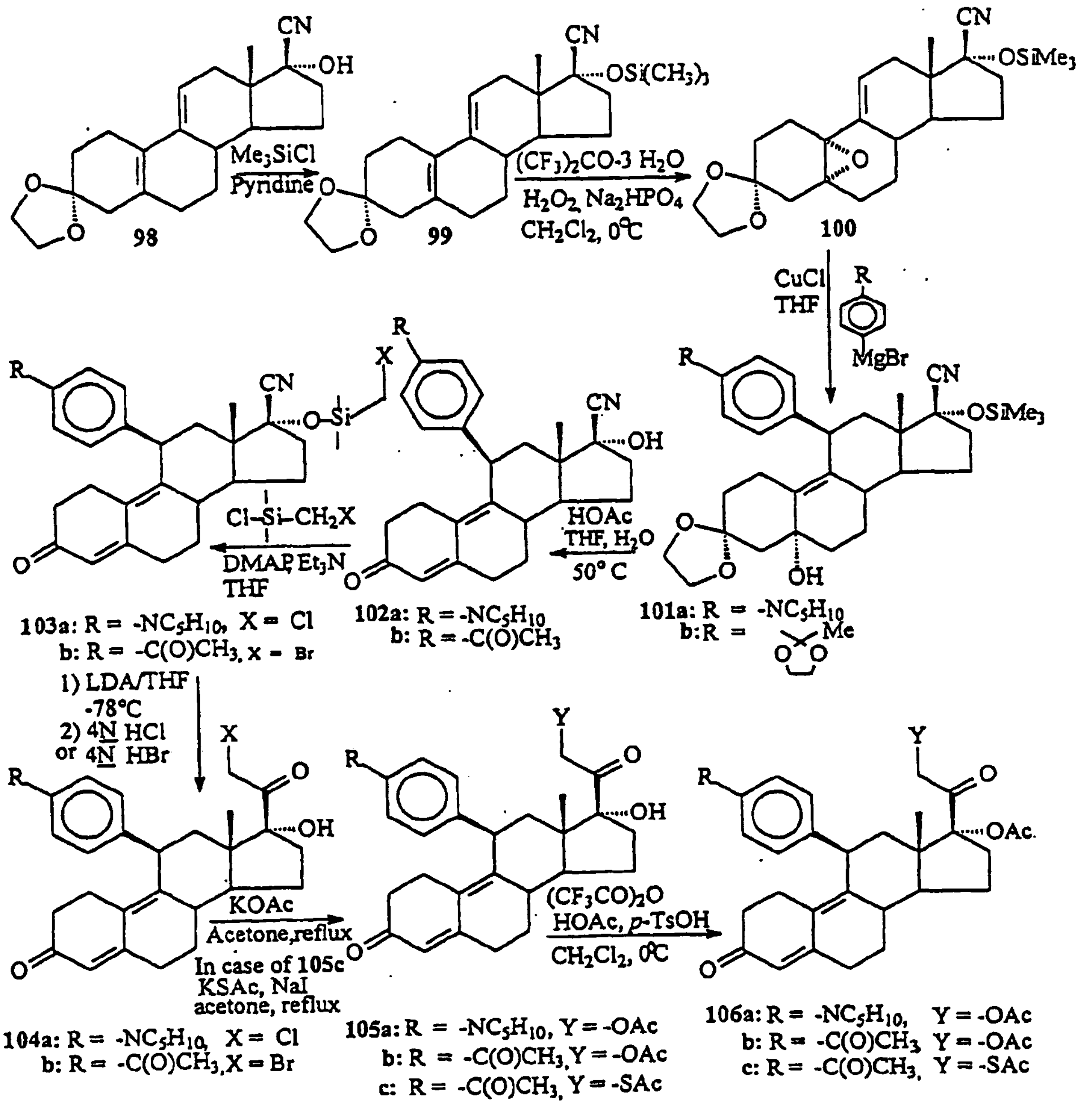


98

99

100

101a: R = -NC$_5$H$_{10}$
b: R = 

102a: R = -NC$_5$H$_{10}$
b: R = -C(O)CH$_3$

103a: R = -NC$_5$H$_{10}$, X = Cl
b: R = -C(O)CH$_3$, X = Br

104a: R = -NC$_5$H$_{10}$, X = Cl
b: R = -C(O)CH$_3$, X = Br

105a: R = -NC$_5$H$_{10}$, Y = -OAc
b: R = -C(O)CH$_3$, Y = -OAc
c: R = -C(O)CH$_3$, Y = -SAc

106a: R = -NC$_5$H$_{10}$, Y = -OAc
b: R = -C(O)CH$_3$, Y = -OAc
c: R = -C(O)CH$_3$, Y = -SAc

Figure 7

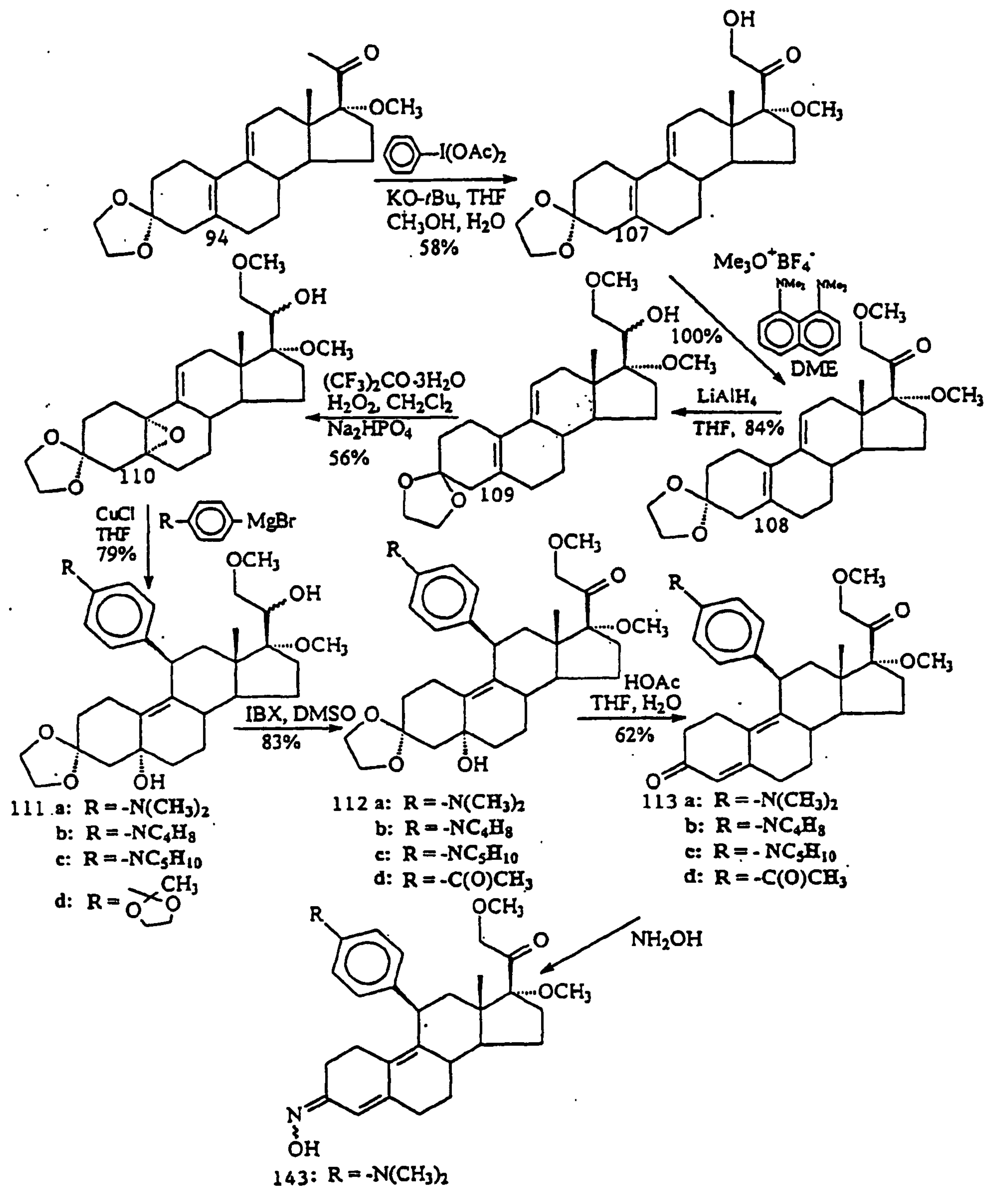
94
I(OAc)2
KO-tBu, THF
CH3OH, H2O
58%
107
Me3O+BF4-
DME
100%
108
LiAlH4
THF, 84%
109
(CF3)2CO·3H2O
H2O2, CH2Cl2
Na2HPO4
56%
110
CuCl
THF
79%
R-
-MgBr
111 a: R = -N(CH3)2
b: R = -NC4H8
c: R = -NC5H10
d: R =
IBX, DMSO
83%
112 a: R = -N(CH3)2
b: R = -NC4H8
c: R = -NC5H10
d: R = -C(O)CH3
HOAc
THF, H2O
62%
113 a: R = -N(CH3)2
b: R = -NC4H8
c: R = - NC5H10
d: R = -C(O)CH3
NH2OH
143: R = -N(CH3)2

Figure 8

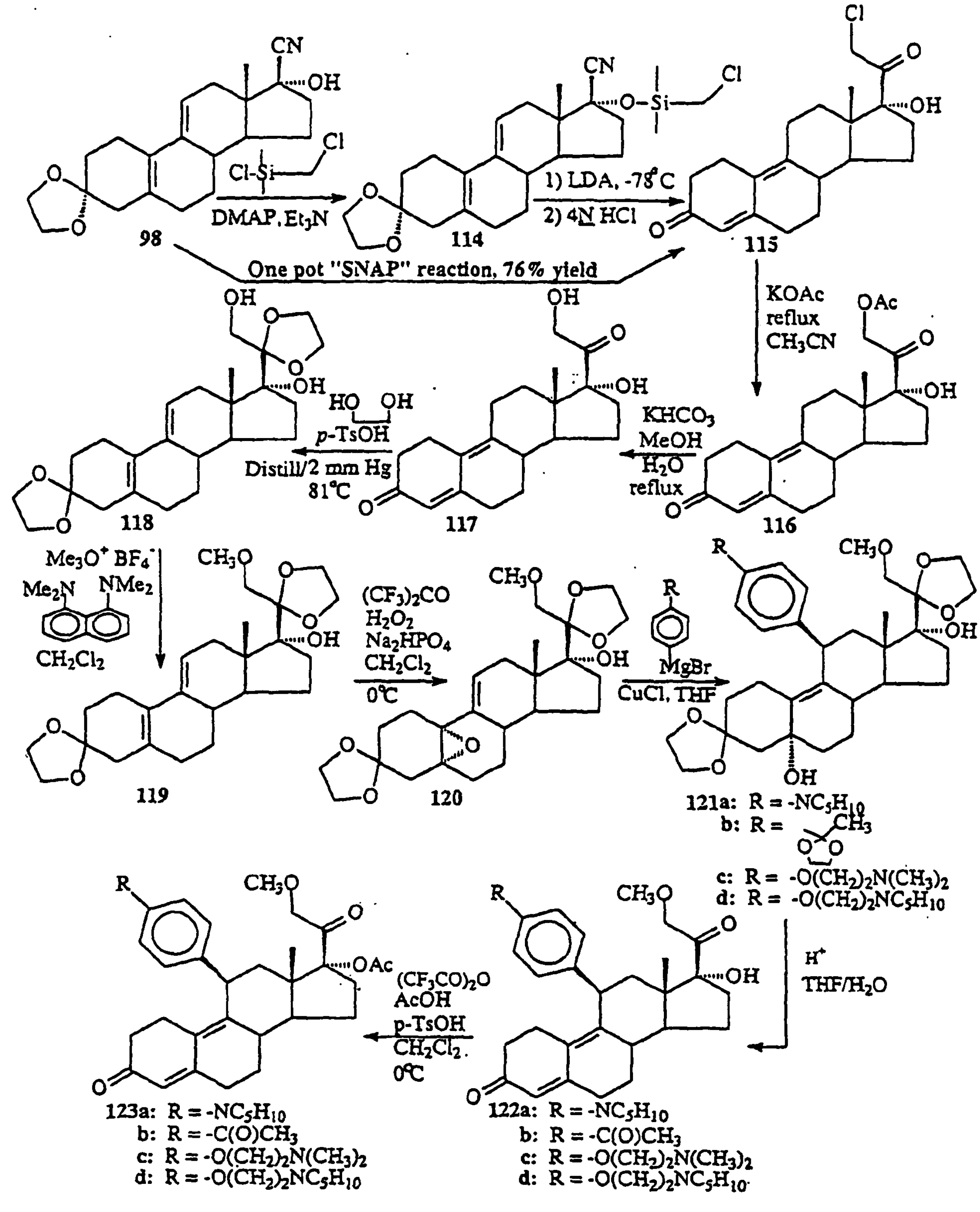
98
DMAP, Et3N
114
1) LDA, -78°C
2) 4N HCl
115
One pot "SNAP" reaction, 76% yield
KOAc
reflux
CH3CN
116
KHCO3
MeOH
H2O
reflux
117
HO OH
p-TsOH
Distill/2 mm Hg
81°C
118
Me3O+ BF4-
CH2Cl2
119
(CF3)2CO
H2O2
Na2HPO4
CH2Cl2
0°C
120
MgBr
CuCl, THF
121a: R = -NC5H10
b: R =
c: R = -O(CH2)2N(CH3)2
d: R = -O(CH2)2NC5H10
H+
THF/H2O
122a: R = -NC5H10
b: R = -C(O)CH3
c: R = -O(CH2)2N(CH3)2
d: R = -O(CH2)2NC5H10
(CF3CO)2O
AcOH
p-TsOH
CH2Cl2
0°C
123a: R = -NC5H10
b: R = -C(O)CH3
c: R = -O(CH2)2N(CH3)2
d: R = -O(CH2)2NC5H10

Figure 9

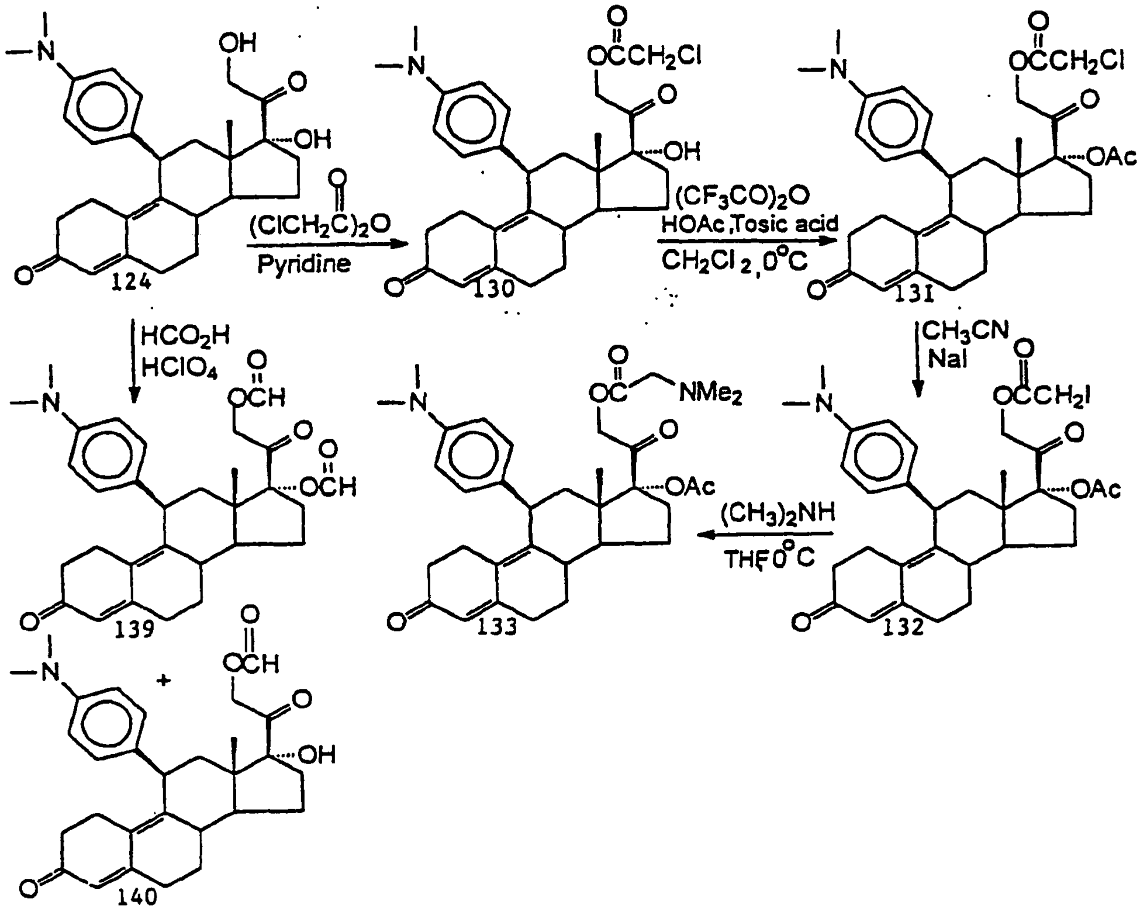
124
(ClCH2C)2O
Pyridine
130
(CF3CO)2O
HOAc,Tosic acid
CH2Cl 2, 0°C
131
CH3CN
NaI
132
(CH3)2NH
THF 0°C
133
HCO2H
HClO4
139
+
140

Figure 10

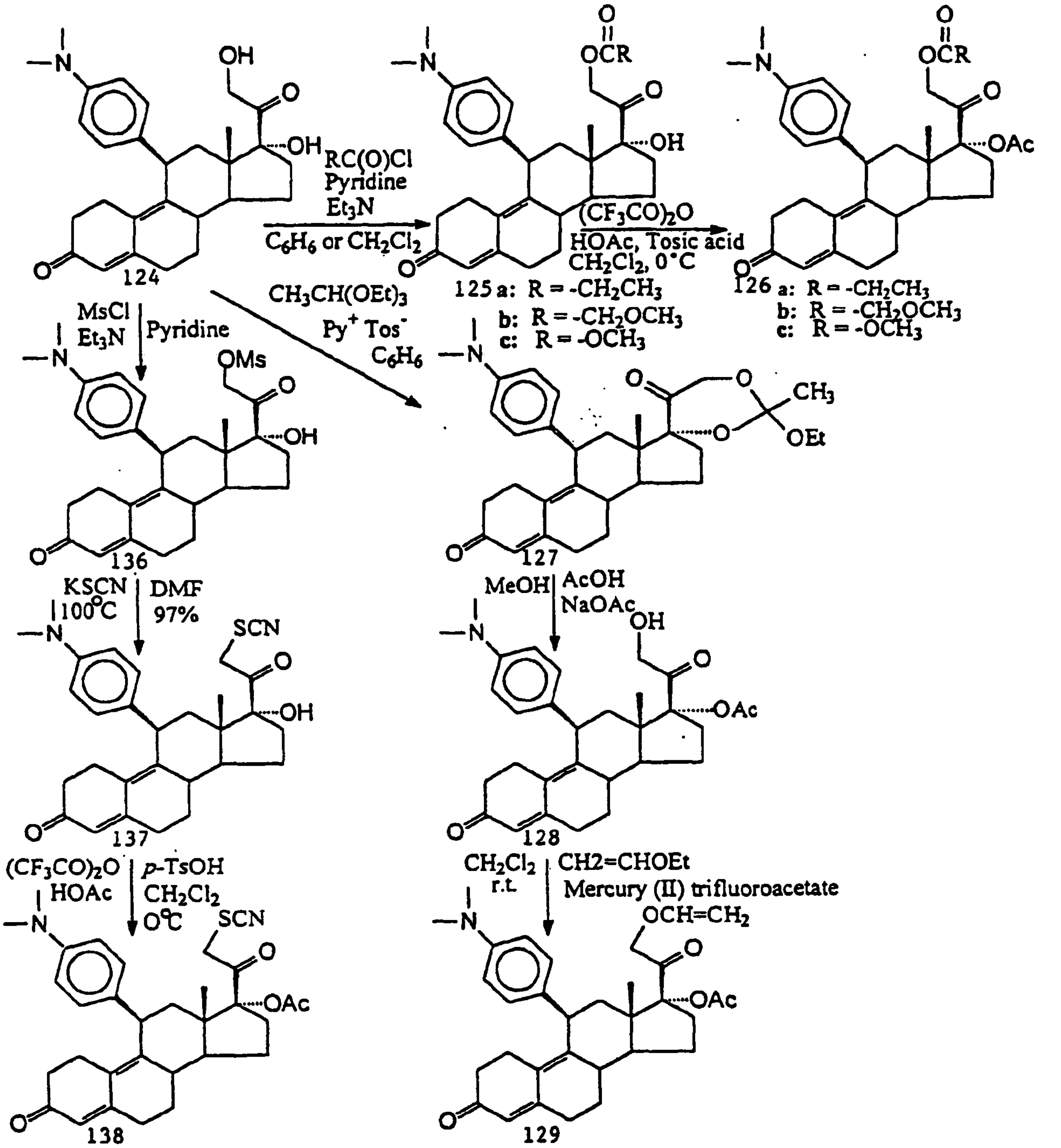
RC(O)Cl
Pyridine
Et3N
C6H6 or CH2Cl2
(CF3CO)2O
HOAc, Tosic acid
CH2Cl2, 0°C
124
125 a: R = -CH2CH3
b: R = -CH2OCH3
c: R = -OCH3
126 a: R = -CH2CH3
b: R = -CH2OCH3
c: R = -OCH3
MsCl
Et3N
Pyridine
CH3CH(OEt)3
Py+ Tos-
C6H6
136
127
KSCN
100°C
DMF
97%
MeOH
AcOH
NaOAc
137
128
(CF3CO)2O
HOAc
p-TsOH
CH2Cl2
0°C
CH2Cl2
r.t.
CH2=CHOEt
Mercury (II) trifluoroacetate
138
129

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 38486
- US 4092693 A, Livingston, D.A.
- US 4977255 A, Livingston, D.A.


### Non-patent literature cited in the description


- **G. TEUTSCH.** Adrenal Steroid Antagonism. Walter de Gruyter and Co, 1984, 43-75
- *IPPF Medical Bulletin,* 1986, vol. 20 (5
- **NIEMAN et al.** *J. Clin. Endocrinology Metab.,* 1985, vol. 61, 536
- **HORWITZ.** *Endocrinology,* 1985, vol. 116, 2236
- **HEIKINHEIMO et al.** *J. Steroid Biochem.,* 1987, vol. 26, 279
- **KETTEL, L.M. et al.** *Fertil Steril,* vol. 56, 402-407
- **MURPHY, A.A. et al.** *Fertil Steril,* vol. 6, 3761-766
- **GROW, D.R. et al.** *J. Clin. Endocrinol. Metab.,* vol. 81, 1933-1939
- **MURPHY, A.A. et al.** *J. CLIN. ENDOCRINOL. METAB.*
- **MURPHY, A.A. et al.** *J. Clin. Endocrinol. Metab.,* vol. 76, 513-517
- **BROGDEN, R.N. et al.** *Drugs,* vol. 45, 384-409
- **BROGDEN, R.N. et al.** *J. CLIN. ENDOCRINOL. METAB.*
- **POISSON, M. et al.** *J. Neurooncol.,* vol. 1, 179-189
- **CARROLL, R.S. et al.** *Cancer Res.,* vol. 53, 1312-1316
- **MAHAJAN, D.K. ; LONDON, S.N.** *Fertil Steril,* 1997, vol. 68, 967-976
- **ROCHEFORT, H.** *Trends in Pharmacol. Sci.,* vol. 8, 126-128
- **HORWITZ, K.B.** *Endocr. Rev.,* 1992, vol. 13, 146-163
- **WOOD, A.J.J.** *N. engl. J. Med.,* 1993, vol. 329, 404-412
- **ULMANN, A. et al.** *Sci. Amer.,* 1990, vol. 262, 42-48
- **REEL, J.R. et al.** *Contraception,* 1998, vol. 58, 129-136
- **ABRAMSON, H.N. et al.** *J. Pharm. Sci.,* 1976, vol. 65, 765-768
- **OLIVETO, E.P. et al.** *J. Am. Chem. Soc.,* 1955, vol. 77, 3564-3567
- **MORIARTY, R.M. et al.** *J. Chem. Soc. Chem. Commun.,* 1981, 641-642
- **VELERIO et al.** *Steroids,* 1995, vol. 60, 268-271
- **LIVINGSTON, D.A. et al.** *J. Am. Chem. Soc.,* 1990, vol. 112, 6449-6450
- **LIVINGSTON, D.A.** *Adv.Med Chem.,* 1992, vol. 1, 137-174
- **CARRUTHERS, N.I. et al.** *J. Org. Chem.,* 1992, vol. 57, 961-965
- **FINCH et al.** *J. Org. Chem.,* 1975, vol. 40, 206-215
- **NUMAZAWA, M. ; NAGAOKA, M.** *J. Chem. Soc. Commun.,* 1983, 127-128
- **NUMAZAWA, M. ; NAGAOKA, M.** *J. Org. Chem.,* 1985, vol. 50, 81-84
- **GLAZIER, E.R.** *J. Org. Chem.,* 1962, vol. 27, 4397-4393
- **WIECHERT, R. ; NEEF, G.** *J. Steroid Biochem.,* 1987, vol. 27, 851-858
- **TEUTSCH, G. et al.** Adrenal Steroid Antagonism. Walter de Gruyter & Co, 1984, 43-75
- **YUR'EV, Y.K. et al.** *Izvest. Akad. Nauk. S.S.S.R., Otdel Khim Nauk,* 1951, 166-171
- **WOLFE, J.P. ; BUCHWALD, S.L.** *J. Org. Chem.,* 1997, vol. 62, 6066-6068
- **VERADRO, G. et al.** *Synthesis,* 1991, 447-450
- **JONES, D.H.** *J. Chem. Soc. (C),* 1971, 132-137
- **DETTY et al.** *J. Am. Chem. Soc.,* 1983, vol. 105, 875-882
- **RAO, P.N. et al.** *Steroids,* 1998, vol. 63, 523-550
- **TEUTSCH, G. ; BELANGER, A.** *Tetrahedron Lett.,* 1979, 2051-2054
- **DESS, D.B. ; MARTIN, J.C.** *J. Org. Chem.,* 1983, vol. 48, 4155-4156
- **FRIGERIO, M. ; SANTAGOSTINO, M.** *Tetrahedron Letters,* 1994, vol. 35, 8019-8022
- **FRIGERIO, M. et al.** *J. Org. Chem.,* vol. 60, 7272-7276

- **MORIARTY et al.** J. Chem. Soc. *Chem. Commun.,* 1981, 641-642
- **DESS et al.** *J Org. Chem.,* 1983, vol. 48, 4155-4156
- **FRIGERIO et al.** *Tet. Letters,* 1994, vol. 35, 8019-8022
- **FRIGERIO et al.** *J. Org. Chem.,* 1995, vol. 60, 7272-7276
- **BLISS, CL.** The Statistics of Bioassay. Academic Press, 1952
- **HOLLISTER, C.** *Nucleic Acids Research,* 1988, vol. 16, 1873-1575
- **MCPHAIL.** *J. Physiol.,* 1934, vol. 83, 145
- **DAO, B. et al.** *Contraception,* 1996, vol. 54, 243-258
- **REEL, J. et al.** *Contraception,* 1998, vol. 58, 129-136
- **BADSTA, M. et al.** *Am. J Obstet. Gynecol.,* 1991, vol. 165, 82-86
- **BAULIEU, E.** Clinical Applications of Mifepristone (RU486) and Other Antiprogestins. National Academy Press, 1993, 72-119